# EUROPEAN PATENT APPLICATION

(11) **EP 4 372 004 A1**
(43) Date of publication of application: **22.05.2024**
(21) Application number: 22841402.5
(22) Date of filing: 13.07.2022
(51) Int. Cl.: C07K 16/18, C12N 15/13, C12N 15/63, C12N 5/10, A61K 39/395, A61P 35/00

(54) **CLDN18.2 BINDING MOLECULES AND USE THEREOF**

(30) Priority: 14.07.2021 CN 202110795793
(71) Applicant: Sanyou Biopharmaceuticals Co., Ltd., Shanghai 201114 (CN)
(72) Inventor: YAN, Xintian, Shanghai 201114 (CN); LANG, Guojun, Shanghai 201114 (CN); TAN, Yongcong, Shanghai 201114 (CN); LIU, Chanjuan, Shanghai 201114 (CN); KONG, Chao, Shanghai 201114 (CN); YAN, Run, Shanghai 201114 (CN); LIU, Yaru, Shanghai 201114 (CN); YAO, Tianen, Shanghai 201114 (CN)
(74) Representative: HGF
(86) International application number: PCT/CN2022/105392
(87) International publication number: WO 2023/284769

(57) **Abstract**

The present invention relates to specific CLDN18.2 binding molecules, and an immunoconjugate and a composition containing the CLDN18.2 binding molecules. The present invention further relates to a nucleic acid encoding the CLDN18.2 binding molecules, a host cell containing same, and a method for preparing the CLDN18.2 binding molecules. Furthermore, the present invention relates to the therapeutic and diagnostic use of the CLDN18.2 binding molecules. In particular, the present invention relates to the combined treatment of the CLDN18.2 binding molecules with other therapies, such as a therapeutic method or a therapeutic agent.

## Description

The present invention relates to specific CLDN18.2 binding molecules, and an immunoconjugate and a composition containing the CLDN18.2 binding molecules. The present invention further relates to a nucleic acid encoding the CLDN18.2 binding molecules, a host cell containing same, and a method for preparing the CLDN18.2 binding molecules. Furthermore, the present invention relates to the therapeutic and diagnostic use of the CLDN18.2 binding molecules. In particular, the present invention further relates to the combined treatment of the CLDN18.2 binding molecules with other therapies, such as a therapeutic method or a therapeutic agent.

### Background Art

CLDN18 belongs to the members of Claudins protein family and was discovered by Shoichiro Tsukita *et al.* in 1998. It is an important molecule that constitutes the tight junction between epithelial cells, determines the permeability of epithelial cells, and also plays a role in blocking the diffusion of proteins and lipids on the cell membrane surface (Gunzel, D. and A. S. Yu (2013). "Claudins and the modulation of tight junction permeability." Physiol Rev 93(2): 525-569). The human CLDN18 gene has two different exons 1, which undergo alternative splicing after transcription to eventually generate two protein isoforms CLDN18.1 and CLDN18.2 with different sequences only at the N terminus. Both CLDN18 isoform proteins are composed of 261 amino acids and have four transmembrane domains, but they are distributed in different tissues. CLDN18.1 is mainly expressed in lung tissue, and CLDN18.2 is only expressed on differentiated epithelial cells of gastric mucosa, and not expressed on gastric stem cells (Sahin, Ugur et al., "Claudin-18 splice variant 2 is a pan-cancer target suitable for therapeutic antibody development." ClinicalCancer Research14.23 (2008): 7624-7634).

CLDN18.2 is highly expressed in a variety of tumour tissues, such as non-small cell lung cancer (25%), gastric cancer (70%), pancreatic cancer (50%) and esophageal cancer (30%), but almost not expressed in normal tissues (Kumar, V. et al., (2018) "Emerging Therapies in the Management of Advanced-Stage Gastric Cancer." Front Pharmacol 9: 404). Due to the difference in expression between tumour cells and normal tissues, CLDN18.2 has become a very potential target of anti-tumour drugs.

Up to now, among the drugs targeting CLDN18.2, the most advanced one is IMAB362 developed by the German company Ganymed. IMAB362 is a human-mouse chimeric IgG1 monoclonal antibody specifically targeting CLDN18.2, binds to the first extracellular domain of CLDN18.2 expressed on tumour cells and induces tumour cell death through antibody-dependent cell-mediated cytotoxicity (ADCC) and complement-dependent cytotoxicity (CDC). IMAB362 significantly prolongs patient survival compared with standard chemotherapy in a phase II trial of gastric cancer (the survival time for standard chemotherapy is 8.4 months; while the survival time of IMAB362 treatment is 13.2 months). The therapeutic effect of IMAB362 is more obvious in patients with high Claudin18.2 expression.

Although there are monoclonal antibody drugs targeting the CLDN18.2 target under current clinical research, the monoclonal antibody (150 kD) has a large molecular weight and is difficult to penetrate tissue, resulting in a low effective concentration in the tumour area and poor therapeutic effect. As a therapeutic agent, there is still an urgent need to continue to develop small molecule antibodies targeting the CLDN18.2 target.

Single domain antibodies (sdAbs) (e.g., nanobodies) are currently the smallest antibody molecules, and their molecular weight is 1/10 of ordinary antibodies. In addition to the antigenic reactivity of monoclonal antibodies, single domain antibodies also possess some unique functional properties. For example, they usually exhibit higher solubility, good thermal stability, tissue penetration, and resistance to degradation of papain; in addition, single domain antibodies can be expressed, in a quite high expression amount, in a plurality of host cells such as yeast, plant and mammalian cells, which endows them with very great cost advantages. Therefore, in the prior art, it is hoped to develop new single domain antibodies (sdAbs) that binds to CLDN18.2 with higher affinity.

### Summary of the Invention

Through intensive research, the inventors have developed a class of CLDN18.2-binding molecules containing a single domain antibody (sdAb) portion that specifically recognizes CLDN18.2, the CLDN18.2-binding molecules are capable of
(1) binding to CLDN18.2, such as human CLDN18.2, with high affinity, for example, the EC₅₀ of the binding between the CLDN18.2 binding molecule and CLDN18.2 on a cell surface is about 0.1 µg/mL to about 10 µg/mL, preferably, about 0.1 µg/mL to about 1 µg/mL;
(2) specifically binding to CLDN18.2 and not binding to CLDN18.1; and
(3) killing CLDN18.2-positive cancer cells through antibody-dependent cell-mediated cytotoxicity and/or complement-dependent cytotoxicity.

Therefore, in a first aspect, the present invention provides a CLDN18.2 binding molecule, which comprises at least one single domain antibody (sdAb) portion that specifically binds to CLDN18.2, and the sdAb portion comprises three complementarity determining regions, namely CDR1, CDR2 and CDR3, respectively, wherein:
(a) the CDR1 comprises the amino acid sequence of SEQ ID NO: 1, or a variant with 1 or 2 amino acid changes in the amino acid sequence of SEQ ID NO: 1;
(b) the CDR2 comprises the amino acid sequence of SEQ ID NO: 2, or a variant with 1 or 2 amino acid changes in the amino acid sequence of SEQ ID NO: 2; and
(c) the CDR3 comprises the amino acid sequence of SEQ ID NO: 3, or a variant with 1 or 2 amino acid changes in the amino acid sequence of SEQ ID NO: 3,
in which the amino acid change is amino acid addition, amino acid deletion or conservative amino acid substitution, preferably, the sdAb portion is a camelid VHH, a partially humanized or fully humanized VHH, or a chimeric VHH.

In some embodiments, the sdAb portion of the CLDN18.2 binding molecule of the present invention comprises: CDR1 comprising the amino acid sequence SEQ ID NO: 1, CDR2 comprising the amino acid sequence SEQ ID NO: 2 and CDR3 comprising the amino acid sequence SEQ ID NO: 3.

In some embodiments, the sdAb portion of the CLDN18.2 binding molecule of the present invention comprises
(i) the amino acid sequence of SEQ ID NO: 4 or 5; or
(ii) an amino acid sequence having at least 85%, 90%, 91%, 92%, 93%, 94%, 95%, 96%, 97%, 98% or 99% identity to the amino acid sequence of SEQ ID NO: 4 or 5.

In some embodiments, the CLDN18.2 binding molecule of the present invention is linked to another protein domain at the N-terminus or C-terminus of the sdAb portion, for example to the Fc region of an immunoglobulin (for example to the Fc region from an IgG, such as IgG1, IgG2, IgG3 or IgG4), or for example to a fluorescent protein.

In some embodiments, the CLDN18.2 binding molecule of the present invention is a bispecific or multispecific antibody, preferably, the bispecific antibody molecule specifically binds to CLDN18.2 molecule and a second target protein, the second target protein is selected from, for example:
(1) a tumour-specific antigen or a tumour-associated antigen, such as epidermal growth factor receptor (EGFR1), HER2/neu, CD20, insulin-like growth factor receptor (IGF-1R), carcinoembryonic antigen, prostate-specific membrane antigen (PSMA), Mucin-1, CD30, CD33, CD137, cMet or angiopoietin-2 (Ang-2);
(2) an immune checkpoint molecule of an immune cell, for example, PD1, CTLA-4, TIM-3, or LAG-3;
(3) an immune costimulatory molecule of an immune cell, for example, OX40, ICOS, TLR2 or CD27;
(4) a cytokine, for example, IL-1, IL-2, IL-7, IL-15 or IL-33.

In a second aspect, the present invention provides a method for preparing the CLDN18.2 binding molecule of the present invention, the method comprises culturing a host cell introduced with a nucleic acid encoding the CLDN18.2 binding molecule of the present invention or an expression vector comprising the nucleic acid under conditions suitable for expression of a nucleic acid encoding the CLDN18.2 binding molecule of the present invention; isolating the CLDN18.2 binding molecule, optionally the method further comprises recovering the CLDN18.2 binding molecule from the host cell.

In a third aspect, the present invention provides an immunoconjugate comprising the CLDN18.2 binding molecule of the present invention and other substances such as a cytotoxic agent.

In a fourth aspect, the present invention provides a pharmaceutical composition comprising the CLDN18.2 binding molecule or immunoconjugate of the present invention, and optionally a pharmaceutical adjuvant material.

In some embodiments, the present invention provides a pharmaceutical composition comprising the CLDN18.2 binding molecule or immunoconjugate of the present invention, and other therapeutic agents, and optionally a pharmaceutical adjuvant material; preferably, the other therapeutic agents are selected from a chemotherapeutic agent, other antibodies (such as an anti-PD-1 antibody or an anti-PD-L1 antibody) and a cytotoxic agent.

In some embodiments, the present invention provides a combined product comprising the CLDN18.2 binding molecule or immunoconjugate of the present invention, and one or more other therapeutic agents, such as a chemotherapeutic agent, a cytotoxic agent and other antibodies, for example, an anti-PD-1 antibody or an anti-PD-L1 antibody.

In a fifth aspect, the present invention provides a method for treating a disease associated with CLDN18.2 in a subject, the method comprises administering to the subject a therapeutically effective amount of the CLDN18.2 binding molecule, the immunoconjugate, the pharmaceutical composition or the combined product of the present invention.

In some embodiments, the disease associated with CLDN18.2 treated by the CLDN18.2 binding molecule, the immunoconjugate, the pharmaceutical composition or the combined product of the present invention is, for example, a cancer that expresses or overexpresses CLDN 18.2.

In a sixth aspect, the present invention provides a kit for detecting CLDN18.2 in a sample, the kit comprises the CLDN18.2 binding molecule of the present invention and is used for performing the following steps:
(a) contacting the sample with the CLDN18.2 binding molecule of the present invention; and
(b) detecting the formation of a complex of the CLDN18.2 binding molecule and CLDN18.2; optionally, the CLDN18.2 binding molecule is detectably labelled,
and therefore, it is determined whether elevated CLDN18.2 expression levels are present in the sample from the subject or individual.

### Brief Description of the Drawings

The following detailed description of the preferred embodiments of the present invention can be better understood when reading in conjunction with the following drawings below. For the purpose of illustrating the present invention, there are shown in the drawings embodiments which are presently preferred. However, it should be understood that the present invention is not limited to the precise arrangements and instrumentalities of the embodiments shown in the drawings.
Fig. 1 shows the SDS-PAGE pattern of anti-CLDN18.2 heavy-chain antibody. The samples are, respectively: heavy-chain antibody NA3SH1-T4, heavy-chain antibody NA3SH1-T4-hVH6, heavy-chain antibody NA3SH1 as a control and reference product IPI (Ipilimumab). Lane marker is an indicator for protein molecular weight.
Fig. 2A shows the monomer detection pattern of heavy-chain antibody NA3SH1-T4 detected by SEC-HPLC.
Fig. 2B shows the monomer detection pattern of heavy-chain antibody NA3SH1-T4-hVH6 detected by SEC-HPLC.
Fig. 2C shows the monomer detection pattern of heavy-chain antibody NA3SH1 as a control detected by SEC-HPLC.
Fig. 3 shows the binding curves of anti-CLDN18.2 heavy-chain antibodies NA3SH1-T4 and NA3SH1-T4-hVH6 and control heavy-chain antibody NA3SH1 to hCLDN18.2-HKE293 cells. MFI represents the average fluorescence intensity.
Fig. 4 shows the binding curves of anti-CLDN18.2 heavy-chain antibodies NA3SH1-T4 and NA3SH1-T4-hVH6 and control heavy-chain antibody NA3SH1 to hCLDN18.2-NUGC4 cells. MFI represents the average fluorescence intensity.
Fig. 5 shows the binding curves of anti-CLDN18.2 heavy-chain antibodies NA3SH1-T4 and NA3SH1-T4-hVH6 and control heavy-chain antibody NA3SH1 to hCLDN18.2-KATOIII cells. MFI represents the average fluorescence intensity.
Fig. 6 shows the binding positivity rate of anti-CLDN18.2 heavy-chain antibodies NA3SH1-T4 and NA3SH1-T4-hVH6 and control heavy-chain antibody NA3SH1 to hCLDN18.1-HEK293 cells at high concentrations (100 µg/mL).
Fig. 7 shows the antibody-dependent cell-mediated cytotoxicity (ADCC) of anti-CLDN18.2 heavy-chain antibodies NA3SH1-T4 and NA3SH1-T4-hVH6 and control heavy-chain antibody NA3SH1 on hCLDN18.2-HEK293 cells.
Fig. 8 shows the antibody-dependent cell-mediated cytotoxicity (ADCC) of anti-CLDN18.2 heavy-chain antibodies NA3SH1-T4 and NA3SH1-T4-hVH6 and control heavy-chain antibody NA3SH1 on hCLDN18.2-KATOIII cells.
Fig. 9 shows the antibody-dependent cell-mediated cytotoxicity (ADCC) of anti-CLDN18.2 heavy-chain antibodies NA3SH1-T4 and NA3SH1-T4-hVH6 and control heavy-chain antibody NA3SH1 on hCLDN18.2-NUGC4 cells.

### Detailed Description of Embodiments

Unless defined otherwise, all technical and scientific terms used herein have the same meanings as commonly understood by those of ordinary skill in the art to which the present invention pertains. All publications, patent applications, patents and other references mentioned herein are incorporated by reference in its entirety. In addition, the materials, methods and examples described herein are illustrative only and are not intended to be limiting. Other features, objectives and advantages of the present invention will be apparent from the description, the drawings and the appended claims.

### I. Definitions

For interpreting this specification, the following definitions will apply and whenever appropriate, terms used in the singular will also include the plural and vice versa. It is to be understood that the terminology used herein is for the purpose of describing particular embodiments only, and is not intended to be limiting.

The term "about" when used in conjunction with a numerical value is meant to encompass numerical values within a range having a lower limit that is 5% smaller than the specified numerical value and an upper limit that is 5% larger than the specified numerical value.

As used herein, the term "and/or" means any one of optional items or two or more of the optional items.

Unless otherwise indicated, the term "comprise" or "include" when used herein also encompasses situations consisting of the described elements, integers or steps. For example, when referring to an antibody variable region "comprise" a particular sequence, it is intended to also encompass an antibody variable region consisting of that particular sequence.

The term "Claudins" is a type of integrin membrane protein that exists in epithelial and endothelial tight junctions and is an important component of tight junctions. It was discovered by Shoichiro Tsukita *et al.* in 1998. The family has 24 members. The human Claudin 18 gene has two alternative exons 1, resulting in two protein isoforms, Claudin 18.1 (also referred to as "CLDN18.1" herein) and Claudin 18.2 (also referred to as "CLDN18.2" herein). There are only 7 amino acid residue differences between the two isoforms in the approximately 50-amino acid sequence of the first extracellular domain.

There is a significant difference in the expression of Claudin 18.2 in cancer tissues and normal tissues. This may be due to the fact that the CREB binding site in the promoter region of Claudin 18.2 is highly methylated in CpG in normal tissues, while the level of CpG methylation in the process of cell canceration decreases, and then CREB participates in activating the transcription of Claudin18.2.

The terms "CLDN18.2 antibody", "antibody against CLDN18.2", "antibody specifically binding to CLDN18.2", "antibody specifically targeting CLDN18.2" and "antibody specifically recognizing CLDN18.2" as used herein are used interchangeably, and mean antibodies that can specifically bind to the Claudin protein CLDN18.2. Particularly, in some specific embodiments, the terms mean an antibody that specifically binds to human CLDN18.2, particularly an antibody that specifically binds to human CLDN18.2 but does not specifically bind to human CLDN18.1.

The term "antibody" herein is used in the broadest sense, refers to a protein comprising an antigen-binding site, and encompasses natural antibodies and artificial antibodies of various structures, including but not limited to monoclonal antibodies, polyclonal antibodies, multispecific antibodies (such as bispecific antibodies), single chain antibodies, single domain antibodies, intact antibodies, and antibody fragments. Preferably, the antibodies of the present invention are single domain antibodies or heavy-chain antibodies.

The term "antibody fragment" refers to a molecule different from an intact antibody, which comprises a portion of the intact antibody and is capable of binding to an antigen to which the intact antibody binds. Examples of antibody fragments include, but are not limited to, Fv, Fab, Fab', Fab'-SH, F(ab')₂, diabodies, linear antibodies, single chain antibodies (such as scFv), single domain antibodies, bivalent or bispecific antibodies or fragments thereof, camelid antibodies (heavy-chain antibodies), and bispecific or multispecific antibodies formed from antibody fragments.

The term "complementarity determining region" or "CDR region" or "CDR" is a region of an antibody variable domain which is hypervariable in the sequence and forms a structurally defined loop ("hypervariable loop") and/or contains an antigen contact residue ("antigen contact point"). CDRs are mainly responsible for binding to antigenic epitopes, and include, sequentially numbered starting from the N-terminus, CDR1, CDR2 and CDR3. In a given heavy chain variable region amino acid sequence, the precise amino acid sequence boundary of each CDR may be determined by using any one of many well-known antibody CDR assignment systems or a combination thereof, wherein the assignment system includes, for example: Chothia based on the three-dimensional structures of antibodies and the topology of CDR loops (Chothia et al., (1989) Nature 342: 877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (http://imgt.cines.fr/), and the North CDR definition based on affinity propagation clustering using a large number of crystal structures.

Unless otherwise stated, in the present invention, the term "CDR" or "CDR sequence" encompass CDR sequences determined by any one of the above methods.

CDR can also be determined on the basis of having the same AbM numbering position as a reference CDR sequence (such as any sequence of the exemplary CDRs of the present invention). In one embodiment, the position of the CDR of the single domain antibodies of the present invention is determined according to an AbM numbering scheme.

Unless otherwise stated, in the present invention, when referring to residue positions (including heavy chain variable region residues) in antibody variable regions and CDRs, it is referred to as numbering positions according to an AbM numbering system.

Antibodies having different specificity (i.e., different binding sites for different antigens) have different CDRs. However, although CDRs vary from antibody to antibody, only a limited number of amino acid positions within the CDRs are directly involved in antigen binding. Using at least two of the Kabat, Chothia, AbM, IMGT, and Contact methods, a minimal overlap region can be determined, thereby providing a "minimal binding unit" for antigen binding. The minimal binding unit may be a sub-portion of a CDR. As will be apparent to a person skilled in the art, the residues of the remainder of the CDR sequence can be determined by the structure of an antibody and by protein folding. Therefore, the present invention also takes variants of any of the CDRs given herein. For example, in a variant of a CDR, the amino acid residue of the minimal binding unit can remain unchanged, while the remaining CDR residues defined according to Kabat or Chothia or AbM or IMGT or Contact can be replaced with conserved amino acid residues.

The term "single domain antibody" generally refers to an antibody in which a single variable domain (e.g., a heavy chain variable domain (VH) or a light chain variable domain (VL), a heavy chain variable domain derived from a camelid heavy-chain antibody, and a VH-like single domain (v-NAR) derived from fish IgNAR) confers antigen binding. That is, the single variable domain does not need to interact with another variable domain to recognize the target antigen. Examples of the single domain antibody include those derived from camelid (llamas and camels) and cartilaginous fish (e.g., nurse sharks) (WO2005/035572). The single domain antibody derived from Camelidae, also referred to in this application as VHH, consists of only one heavy chain variable region, is an antibody comprising only one chain FR4-CDR3-FR3-CDR2-FR2-CDR1-FR1 from the C-terminus to the N-terminus, and is also called "nanobody". The single domain antibody is the smallest unit currently known that can bind to a target antigen.

The term "heavy-chain antibody (hcAb)" refers to an antibody without a light chain, which may comprise VH-CH2-CH3, or comprise VH-CH1-CH2-CH3, comprise VHH-CH2-CH3, *etc.* from N-terminus to C-terminus; Homodimers can be formed, such as dimeric heavy-chain antibodies without light chains. The heavy-chain antibody may comprise the VH from a standard antibody or the VHH from a single domain antibody. In one embodiment, the heavy-chain antibody of the present invention comprises the VHH of a single domain antibody.

As used herein, the term "multispecific antibody" refers to an antibody having at least two antigen-binding sites, each of which binds a different epitope of the same antigen or a different epitope of a different antigen. The multispecific antibody is an antibody with binding specificities for at least two different antigen epitopes. In one embodiment, provided herein is a multispecific antibody having binding specificities for a first antigen and a second antigen, also referred to as "bispecific antibody."

The term "effector function" refers to those biological activities attributed to the Fc region of an immunoglobulin that vary with immunoglobulin isoform. Examples of immunoglobulin effector functions include: C1q binding and complement-dependent cytotoxicity (CDC), Fc receptor binding, antibody-dependent cell-mediated cytotoxicity (ADCC), antibody-dependent cell-mediated phagocytosis (ADCP), cytokine secretion, immune complex-mediated antigen uptake by antigen-presenting cells, down regulation of cell surface receptors (e.g., B cell receptors), and B cell activation.

The term "antibody-dependent cell-mediated cytotoxicity (ADCC)" is one of the major mechanisms by which certain cytotoxic effector cells, such as natural killer (NK) cells, mediate killing on target cells and foreign host cells. The Fc region of the antibody binds to, for example, the Fc receptor FcγRIIIA (i.e., CD16a) expressed on NK cells and then activates NK cells to exert the ADCC effect. CD16a is a member of the transmembrane receptor of the immunoglobulin superfamily. According to the allelic polymorphism difference at position 158 from its N-terminus, CD16a has differential expression of valine or phenylalanine at position 158, resulting in the presence of CD16a-158V/V (approximately 15%), CD16a-158V/F (approximately 25%) and CD16a-158F/F (approximately 60%) subtypes in the population.

The term "complement-dependent cytotoxicity (CDC)" refers to lysis of a target cell in the presence of a complement. Activation of the classical complement pathway is initiated by the binding of a first component (C1q) of the complement system to an antibody (an appropriate subclass) binding to its cognate antigen. To evaluate the complement activation, a CDC assay may be performed by using, for example, a method as described in Gazzano-Santoro et al., J. Immunol. Methods 202:163 (1996).

The term "chimeric antibody" is an antibody molecule in which (a) the constant region or a portion thereof is altered, replaced, or exchanged such that the antigen binding site is associated with a constant region belonging to different or changed classes, having different or changed effector functions and/or belonging to different or changed species or a completely different molecule (e.g., enzymes, toxins, hormones, growth factors, drugs) that gives a chimeric antibody new properties; or (b) the variable region or a portion thereof is altered, replaced or exchanged with a variable region with a different or changed antigen specificity. For example, a camel antibody can be modified by replacing its constant region with a constant region from a human immunoglobulin. Due to the replacement with a human constant region, the chimeric antibody can retain its specificity in recognizing the antigen while having reduced antigenicity in humans as compared to the original camel antibody.

The term "humanized antibody" refers to a chimeric antibody comprising amino acid residues from non-human CDRs and amino acid residues from human FRs. In some embodiments, all or substantially all CDRs (e.g., CDRs) in a humanized antibody correspond to those in a non-human antibody, and all or substantially all FRs correspond to those in a human antibody. A humanized antibody optionally may comprise at least a portion of an antibody constant region derived from a human antibody. A "humanized form" of an antibody (e.g., a non-human antibody) refers to an antibody that has undergone humanization.

The term "human antibody" refers to an antibody having an amino acid sequence corresponding to that of an antibody produced by human or human cells or derived from a non-human source using human antibody repertoire or other human antibody coding sequences. This definition of a human antibody explicitly excludes a humanized antibody comprising non-human antigen-binding residues.

The term "Fc region" herein is used to define a C-terminal region of an immunoglobulin heavy chain, comprising at least a portion of the constant region. The term includes a natural sequence Fc region and a variant Fc region. In certain embodiments, a human IgG heavy chain Fc region can extend from Cys226 or Pro230 to the carboxyl terminus of the heavy chain. However, the C-terminal lysine (Lys447) of an Fc region may or may not be present. Unless otherwise stated, the amino acid residues in an Fc region or a constant region is numbered according to the EU numbering system, also known as the EU index, as described in Kabat et al., Sequences of Proteins of Immunological Interest, 5th Ed. Public Health Service, National Institutes of Health, Bethesda, MD, 1991.

The term "variable region" or "variable domain" refers to an antibody heavy or light chain domain that is involved in the binding of an antibody to an antigen. The heavy and light chain variable domains of a natural antibody generally have similar structures, with each domain comprising four conserved framework regions (FRs) and three complementarity determining regions (CDRs). (See, for example, Kindt et al., Kuby Immunology, 6th ed., W. H. Freeman and Co., page 91 (2007)). A single VH or VL domain may be sufficient to confer antigen-binding specificity.

As used herein, the term "binding" or "specific binding" means that the binding is selective for an antigen and can be distinguished from unwanted or non-specific interactions. The ability of an antibody to bind to a particular antigen can be determined by enzyme-linked immunosorbent assay (ELISA), SPR or biolayer interferometry or other conventional binding assays known in the art.

The term "immune checkpoint molecule" refers to a class of inhibitory signalling molecules present in the immune system that avoid tissue damage by regulating the persistence and intensity of immune responses in peripheral tissues and participate in maintaining tolerance to self-antigens (Pardoll DM., The blockade of immune checkpoints in cancer immunotherapy. Nat Rev Cancer, 2012, 12(4): 252-264). Research has found that one of the reasons why tumour cells are able to evade the immune system in the body and proliferate out of control is the use of inhibitory signalling pathways of immune checkpoint molecules, thereby inhibiting the activity of T lymphocytes, making T lymphocytes unable to effectively exert their killing effect on tumours (Yao S, Zhu Y and Chen L., Advances in targeting cell surface signalling molecules for immune modulation. Nat Rev Drug Discov, 2013, 12(2):130-146). Immune checkpoint molecules include, but are not limited to, programmed cell death protein 1 (PD-1), PD-L2, LAG-3, and TIM-3.

The term "costimulatory molecule" refers to a corresponding binding partner on a T cell that specifically binds to a costimulatory ligand to mediate a costimulatory response (such as, but not limited to, proliferation) of the T cell. Costimulatory molecules are cell surface molecules other than antigen receptors or ligands thereof that contribute to an effective immune response. Costimulatory molecules include, but are not limited to, MHC class I molecules, TNF receptor proteins, immunoglobulin-like proteins, cytokine receptors, integrins, signalling lymphocyte activating molecules (SLAM proteins), activated NK cell receptors, OX40, CD40, GITR, 4-1BB (*i.e.*, CD137), CD27, and CD28. In some embodiments, the "costimulatory molecule" is CD28, OX40, GITR, 4-1BB (*i.e.*, CD137), and/or CD27.

The term "cytokine" is a generic term for proteins released by a cell population to act as an intercellular mediator on another cell. Examples of such cytokines are lymphokines, monokines, interleukins (ILs), such as IL-1, IL-1α, IL-2, IL-3, IL-4, IL-5, IL-6, IL- 7, IL-8, IL-9, IL-11, IL-12, IL-15; tumour necrosis factors such as TNF-α or TNF-β; and other polypeptide factors including γ-interferon.

The term "immunoconjugate" refers to an antibody conjugated to one or more other substances (including but not limited to a cytotoxic agent or a label).

The term "half effective concentration (EC₅₀)" refers to the concentration of a drug, antibody or toxic agent which induces a response at 50% between the baseline and the maximum after a specific exposure time. In the context of the present application, the unit of EC₅₀ is "µg/mL".

The term "therapeutically effective amount" refers to an amount effective to achieve the desired therapeutic result at a dose and for periods of time desired. The therapeutically effective amount of an antibody or an antibody fragment or a conjugate or composition thereof can vary depending on a variety of factors such as disease state, age, sex and weight of an individual, and the ability of the antibody or antibody moiety to activate a desired response in an individual. A therapeutically effective amount is also an amount in which any toxic or harmful effect of an antibody or an antibody fragment or a conjugate or composition thereof is less than a therapeutically beneficial effect. The "therapeutically effective amount" preferably inhibits measurable parameters (such as tumour growth rate and tumour volume) by at least about 20%, more preferably at least about 40%, even more preferably at least about 50%, 60% or 70% and still more preferably at least about 80% or 90% relative to untreated subjects. The ability of a compound to inhibit a measurable parameter (such as cancer) can be evaluated in an animal model system for predicting the efficacy in a human tumour.

The terms "individual" and "subject" can be used interchangeably, including mammals. Mammals include, but are not limited to, domesticated animals (e.g., cows, sheep, cats, dogs and horses), primates (e.g., humans and non-human primates such as monkeys), rabbits and rodents (e.g., mice and rats). Particularly, the individual or subject is a human.

The terms "tumour" and "cancer" can be used interchangeably herein, and encompass solid tumours and liquid tumours.

The terms "cancer" and "cancerous" refer to the physiological illness in mammals in which cell growth is unregulated.

The term "tumour" refers to all neoplastic cell growth and proliferation, whether malignant or benign, and all pre-cancerous and cancerous cells and tissues. The terms "cancer", "cancerous" and "tumour" are not mutually exclusive when referred to herein.

The term "isolated CLDN18.2 binding molecule" refers to having been separated from components of the natural environment thereof. In some embodiments, the CLDN18.2 binding molecule is purified to more than 95% or 99% purity, as determined by, for example, electrophoresis (e.g., SDS-PAGE, isoelectric focusing (IEF), and capillary electrophoresis) or chromatography (e.g., ion exchange or reversed-phase HPLC, and SEC-HPLC). For a review of methods for evaluating the purity of an antibody, see, for example, Flatman et al., J. Chromatogr. B848: 79-87 (2007).

The term "size-exclusion high-performance liquid chromatography (SEC-HPLC)" is an important method used for antibody standards and quality control. This method mainly separates molecules based on their size or hydrodynamic radius differences. By SEC-HPLC, antibodies can be separated into three main forms: high molecular weight form (HMMS), main peak (mainly antibody monomer) and low molecular weight form (LMMS). Antibody purity can be calculated as the percentage of the main peak area to the sum of all peak areas on the chromatogram. Through the SEC-HPLC method, the percentage of antibody monomers in the preparation product can be measured, giving information on the content of soluble aggregates and shear products.

The term "isolated" nucleic acid refers to a nucleic acid molecule that has been separated from components of the natural environment thereof. An isolated nucleic acid includes a nucleic acid molecule contained in cells that ordinarily contain the nucleic acid molecule, but the nucleic acid molecule is present extrachromosomally or at a chromosomal location that is different from its natural chromosomal location. The "isolated nucleic acid encoding CLDN18.2 binding molecule" refers to one or more nucleic acid molecules encoding the chain or fragment of a CLDN18.2 binding molecule, including such nucleic acid molecules in a single vector or separate vectors, and such nucleic acid molecules present at one or more locations in a host cell.

The calculation of sequence identity between sequences is performed as follows.

To determine the percent identity of two amino acid sequences or two nucleic acid sequences, the sequences are aligned for optimal comparison purposes (for example, gaps can be introduced in either or both of the first and second amino acid sequences or nucleic acid sequences for optimal alignment or non-homologous sequences can be discarded for comparison purposes). In a preferred embodiment, the length of a reference sequence aligned for comparison purposes is at least 30%, preferably at least 40%, more preferably at least 50%, 60% and even more preferably at least 70%, 80%, 90% and 100% of the length of the reference sequence. The amino acid residues or nucleotides at corresponding amino acid positions or nucleotide positions are then compared. When the position in the first sequence is occupied by the same amino acid residue or nucleotide as that at the corresponding position in the second sequence, the molecules are identical at that position.

The sequence comparison and the calculation of percent identity between two sequences can be achieved using a mathematical algorithm. In a preferred embodiment, the percent identity between two amino acid sequences is determined using the Needlema-Wunsch ((1970) J. Mol. Biol. 48:444-453) algorithm which has been incorporated into a GAP program in a GCG software package (available at http://www.gcg.com), and using a Blossum 62 matrix or a PAM250 matrix, a gap weight of 16, 14, 12, 10, 8, 6 or 4 and a length weight of 1, 2, 3, 4, 5 or 6. In another preferred embodiment, the percent identity between two nucleotide sequences is determined using the GAP program in the GCG software package (available at http://www.gcg.com) and using a NWSgapdna.CMP matrix, a gap weight of 40, 50, 60, 70 or 80 and a length weight of 1, 2, 3, 4, 5 or 6. A particularly preferred parameter set (and one parameter set that should be used unless otherwise stated) is a Blossum 62 scoring matrix with a gap penalty of 12, a gap extension penalty of 4 and a gap frameshift penalty of 5.

The percent identity between two amino acid sequences or nucleotide sequences can also be determined using a PAM120 weighted remainder table, with a gap length penalty of 12 and a gap penalty of 4, and using the E. Meyers and W. Miller algorithm ((1989) CABIOS, 4:11-17) that has been incorporated into the ALIGN program (version 2.0).

Additionally or alternatively, the nucleic acid sequences and protein sequences described herein can further be used as "query sequences" to perform searches against public databases, e.g., to identify other family member sequences or related sequences.

The term "transfection" refers to the process of introducing a nucleic acid into eukaryotic cells, particularly mammalian cells. Solutions and techniques for transfection include, but are not limited to, lipofection, and transfection using chemical and physical methods such as electroporation. Many transfection techniques are well-known in the art, and see e.g., Graham et al., 1973, Virology 52:456; Sambrook et al., 2001, Molecular Cloning: A Laboratory Manual; Davis et al., 1986, Basic Methods in Molecular Biology, Elsevier; Chu et al., 1981, Gene 13:197.

The term "fluorescence-activated cell sorting" or "FACS" refers to a specialized type of flow cytometry. It provides a method for sorting a heterogeneous mixture of biological cells into two or more containers, one cell at a time, based upon the specific light scattering and fluorescent characteristics of each cell (FlowMetric. "Sorting Out Fluorescence Activated Cell Sorting". 2017-11-09). Instruments for performing FACS are known to those skilled in the art and are commercially and publicly available. Examples of such instruments include FACS Star Plus, FACScan, and FACSort instruments from Becton Dickinson (Foster City, CA), Epics C from Coulter Epics Division (Hialeah, FL) and MoFlo from Cytomation (Colorado Springs, Colorado).

The term "disease associated with CLDN18.2" refers to any condition induced by or exacerbated by or otherwise associated with increased expression or activity of CLDN18.2 (e.g., human CLDN18.2).

The term "pharmaceutical composition" refers to a composition that is present in a form which allows the active ingredients contained therein to be biologically effective and does not contain additional ingredients that would be unacceptably toxic to a subject to which the pharmaceutical composition is administered.

The term "pharmaceutical adjuvant material" refers to a diluent, an adjuvant (such as Freund's adjuvant (complete and incomplete)), a carrier, an excipient, a stabilizer, *etc.* administered with an active substance.

As used herein, the "treatment" refers to slowing, interrupting, blocking, relieving, stopping, reducing or reversing the progression or severity of an existing symptom, disorder, condition or disease. Desirable effects of treatment include, but are not limited to, preventing occurrence or recurrence of a disease, alleviating symptoms, diminishing any direct or indirect pathological consequences of a disease, preventing metastasis, decreasing the rate of disease progression, ameliorating or palliating disease state, and relieving or improving prognosis. In some embodiments, the antibody molecule of the present invention is used to delay development of a disease or to slow the progression of a disease.

The term "therapeutic agent" described herein encompasses any substance effective in the treatment of tumours (e.g., cancers), including chemotherapeutic agents, cytotoxic agents, other antibodies, small molecule drugs or immunomodulators.

As used herein, the term "chemotherapeutic agent" includes chemical compounds useful in the treatment of cancers, including but not limited to anti-tumour agents, for example, alkylating agents; antimetabolites; antiestrogens; antiandrogens; non-steroidal antiandrogens, etc. Examples of chemotherapeutic agents are those disclosed in WO2015/153513, WO2016/028672 or WO2015/138920.

As used herein, the term "immunomodulator" refers to a natural or synthetic active agent or drug that inhibits or modulates an immune response. The immune response can be a humoral immune response or a cellular immune response. Immunomodulators include inhibitors of immune checkpoint molecules and activators of costimulatory molecules.

As used herein, the term "cytotoxic agent" refers to a substance that inhibits or prevents cell functions and/or causes cell death or destruction. Examples of cytotoxic agents are those disclosed in WO2015/153513, WO2016/028672 or WO2015/138920.

The term "combined product" refers to a fixed or non-fixed combination in the form of a dosage unit or a kit of parts for combined administration in which two or more therapeutic agents may be administered independently at the same time or separately within a certain time interval, especially when such a time interval allow the combined various therapeutic agents to exhibit collaborative effects, e.g., synergistic effects. The term "fixed combination" means that the CLDN18.2 binding molecule of the present invention and a combination partner (e.g., other therapeutic agents, such as anti-PD-1 antibodies or anti-PD-L1 antibodies) are administered simultaneously to a patient in the form of a single entity or dose. The term "non-fixed combination" means that the CLDN18.2 binding molecule of the present invention and a combination partner (e.g., other therapeutic agents, such as anti-PD-1 antibodies or anti-PD-L1 antibodies) are administered to a patient simultaneously, concurrently, or sequentially as separate entities, without specific time limits, wherein such administration provides therapeutically effective levels of both therapeutic agents in the patient. The latter also applies to cocktail therapy, e.g., the administration of three or more therapeutic agents. In a preferred embodiment, the pharmaceutical combination is a non-fixed combination.

The term "combination therapy" or "combined therapy" refers to the administration of two or more therapeutic agents to treat cancers as described in the present disclosure. Such an administration involves co-administration of the therapeutic agents in a substantially simultaneous manner, for example, in a single capsule having a fixed ratio of the active ingredients. Alternatively, such an administration involves co-administration or separate or sequential administration of the individual active ingredients in several or in separate containers (for example, tablets, capsules, powders and liquids). Powders and/or liquids can be reconstituted or diluted to the desired dosage prior to administration. In some embodiments, the administration also includes administering each type of therapeutic agent at approximately the same time, or at different times in a sequential manner. In either case, the treatment regimen would provide for the beneficial effects of the pharmaceutical composition in treating the disorders or conditions described herein.

The term "vector" when used herein refers to a nucleic acid molecule capable of propagating another nucleic acid to which it is linked. The term includes the vector as a self-replicating nucleic acid structure and the vector incorporated into the genome of a host cell into which it has been introduced. Some vectors are capable of directing the expression of nucleic acids to which they are effectively linked. Such vectors are referred to herein as "expression vectors".

The term "host cell" refers to a cell into which an exogenous polynucleotide is introduced, including the progeny of such cells. Host cells include "transformant" and "transformed cell," which include primary transformed cells and progeny derived therefrom regardless of the number of passages. The progeny may be not completely identical to the parent cell in terms of nucleic acid content, but may contain mutations. The mutant progeny that has the same function or biological activity screened or selected in the original transformed cell is included herein. Host cells are any type of cellular system that can be used to produce the antibody molecule of the present invention, including eukaryotic cells such as mammalian cells, insect cells and yeast cells; and prokaryotic cells, for example, E. coli cells. Host cells include cultured cells, and also include cells inside transgenic animals, transgenic plants or cultured plant tissues or animal tissues.

The term "subject/patient sample" refers to a collection of cells, tissues or bodily fluids obtained from a patient or subject. The source of a tissue or cell sample may be a solid tissue, such as from a fresh, frozen and/or preserved organ, a tissue sample, a biopsy sample or a puncture sample; blood or any component of blood; body fluids, such as cerebrospinal fluid, amniotic fluid (liquor amnii), peritoneal fluid (ascites) or interstitial fluid; and cells from any time of pregnancy or development of a subject. Tissue samples may contain compounds that are not naturally intermixed with tissues in nature, such as preservatives, anticoagulants, buffers, fixatives, nutrients and antibiotics. Examples of tumour samples herein include, but are not limited to, tumour biopsies, fine needle aspirates, bronchial lavage fluid, pleural fluid (hydrothorax), sputum, urine, surgical specimens, circulating tumour cells, blood serum, blood plasma, circulating plasma proteins, ascites, primary cell cultures or cell lines derived from tumours or exhibiting tumour-like properties, and preserved tumour samples such as formalin-fixed, paraffin-embedded or frozen tumour samples.

The term "package insert" is used to refer to the instructions for use typically included in commercial packages of therapeutic products that contain information regarding indications, usage, dosage, administration, combination therapies, contraindications and/or warnings involving the use of such therapeutic products.

### II. CLDN18.2 binding molecules of the present invention

The CLDN18.2 binding molecules of the present invention comprises at least one single domain antibody (sdAb) portion that specifically binds to CLDN18.2 but does not bind or substantially does not bind to CLDN18.1, and the sdAb portion comprises, from N-terminus to C-terminus, three complementarity determining regions, namely CDR1, CDR2 and CDR3, wherein:
(a) the CDR1 comprises the amino acid sequence of SEQ ID NO: 1, or a variant with 1 or 2 amino acid changes in the amino acid sequence of SEQ ID NO: 1;
(b) the CDR2 comprises the amino acid sequence of SEQ ID NO: 2, or a variant with 1 or 2 amino acid changes in the amino acid sequence of SEQ ID NO: 2; and
(c) the CDR3 comprises the amino acid sequence of SEQ ID NO: 3, or a variant with 1 or 2 amino acid changes in the amino acid sequence of SEQ ID NO: 3,
wherein the amino acid change is an amino acid addition, amino acid deletion or conservative amino acid substitution.

In some embodiments, the CLDN18.2 binding molecules of the present invention bind to mammalian CLDN18.2, such as human CLDN18.2. For example, the CLDN18.2 binding molecules of the present invention specifically bind to the extracellular domain 1 (ECD1) of human CLDN18.2.

In some embodiments, the CLDN18.2 binding molecules of the present invention have one or more of the following properties:
(1) binding to CLDN18.2, such as human CLDN18.2, with high affinity, for example, the EC₅₀ of the binding between the CLDN18.2 binding molecule and CLDN18.2 on a cell surface is about 0.1 µg/mL to about 10 µg/mL, preferably, about 0.1 µg/mL to about 1 µg/mL;
(2) specifically binding to CLDN18.2 and not binding to CLDN18.1;
(3) killing CLDN18.2-positive cancer cells through antibody-dependent cellular cytotoxicity and/or complement-dependent cytotoxicity.

In some embodiments, the sdAb portion of the CLDN18.2 binding molecule of the present invention comprises: CDR1 comprising the amino acid sequence SEQ ID NO: 1, CDR2 comprising the amino acid sequence SEQ ID NO: 2 and CDR3 comprising the amino acid sequence SEQ ID NO: 3.

In some embodiments, the CLDN18.2 binding molecule of the present invention comprises at least one single domain antibody (sdAb) portion that specifically binds to CLDN18.2, and the sdAb portion is VHH. In some embodiments, the VHH comprises or consists of the following sequences:
(i) the amino acid sequence of SEQ ID NO: 4 or 5;
(ii) an amino acid sequence having at least 85%, 90%, 91%, 92%, 93%, 94%, 95%, 96%, 97%, 98% or 99% identity to the amino acid sequence of SEQ ID NO: 4 or 5; or
(iii) comprising or consisting of an amino acid sequence having one or more (preferably no more than 10, more preferably no more than 6, 5, 4, 3, 2, 1) amino acid changes (preferably amino acid substitution, more preferably amino acid conservative substitution) compared to the amino acid sequence of SEQ ID NO: 4 or 5, preferably, the amino acid changes do not occur in the CDR region.

In some embodiments, the CLDN18.2 binding molecules of the present invention comprise at least one single domain antibody (sdAb) portion that specifically binds to CLDN18.2, and the sdAb portion is a partially humanized or fully humanized VHH or a chimeric VHH. Compared with camelid VHH_{S}, the partially humanized or fully humanized VHHs or chimeric VHHs of the present invention have reduced human anti-camelid antibody responses to humans, improving the safety of antibody application; and are affinity matured VHHs.

In some embodiments, the CLDN18.2 binding molecule of the present invention is linked to the Fc region of an immunoglobulin at the N- or C-terminus of the sdAb portion of the CLDN18.2 binding molecule, optionally via an amino acid linker, e.g., via an amino acid linker having a length between 1 and 20 amino acids. In some embodiments, at least 90% of the amino acid linkers are glycine and/or serine. In some embodiments, the Fc region is from IgG, such as IgG1, IgG2, IgG3 or IgG4. In some embodiments, the Fc region is from IgG1. In some embodiments, the Fc region is from human IgG1.

In some embodiments of the present invention, the amino acid change described herein includes an amino acid substitution, amino acid insertion or amino acid deletion. Preferably, the amino acid change described herein is a substitution of an amino acid, preferably a conservative substitution.

In preferred embodiments, the amino acid change of the present invention occurs in a region outside a CDR (such as in an FR). More preferably, the amino acid change of the present invention occurs in a region outside the VHH. In some embodiments, the substitution is a conservative substitution. The term "conservative substitution" refers to the substitution of an amino acid by another amino acid within the same class, such as the substitution of an acidic amino acid by another acidic amino acid, the substitution of a basic amino acid by another basic amino acid, or the substitution of a neutral amino acid by another neutral amino acid. Exemplary substitutions are shown in Table 1 below:

**Table 1**

| Original Residues | Exemplary substitutions | Preferred substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

In some embodiments, the CLDN18.2 binding molecules provided herein are altered to increase or decrease the extent to which they are glycosylated. Addition or deletion of glycosylation sites to the CLDN18.2 binding molecules is conveniently accomplished by altering the amino acid sequence to create or remove one or more glycosylation sites. When the CLDN18.2 binding molecule comprises an Fc region, the saccharides linked to the Fc region can be changed. In some applications, it may be useful to remove unwanted glycosylation site modifications, for example to remove fucose modules to increase antibody-dependent cell-mediated cytotoxicity (ADCC) function (see Shield et al., (2002) JBC 277:26733). In other applications, galactosylation modifications may be performed to modulate complement-dependent cytotoxicity (CDC). In certain embodiments, one or more amino acid modifications can be introduced into the Fc regions of the CLDN18.2 binding molecules provided herein, thereby creating Fc region variants in order to enhance, for example, the effectiveness of the CLDN18.2 binding molecules of the present invention in treating cancers.

In some embodiments, the CLDN18.2 binding molecules of the present invention are in the form of bispecific or multispecific antibody molecules. In one embodiment, the bispecific antibody molecule binds to CLDN18.2 and PD-1. The multispecific antibody molecule may, for example, be a trispecific antibody molecule comprising a first binding specificity for CLDN18.2 and a second and third binding specificity for one or more of: PD-1, PD-L1, 4-1BB, OX40 or LAG-3.

### III. Immunoconjugates

The present invention also relates to the CLDN18.2 binding molecules of the present invention conjugated to other substances ("immunoconjugates"). In some embodiments, the other substance is, for example, a therapeutic agent (such as a cytotoxic agent). Cytotoxic agents include any agents that are harmful to cells. Examples of cytotoxic agents (such as chemotherapeutic agents) suitable for forming immunoconjugates are known in the art. For example, the cytotoxic agents include, but are not limited to: radioisotopes; growth inhibitors; toxins such as small molecule toxins, or enzymatically active toxins of bacterial, fungal, plant or animal origin, including fragments and/or variants thereof; and various known anti-tumour or anti-cancer agents.

With regard to examples of cytotoxic agents (such as chemotherapeutic agents) suitable for forming immunoconjugates, see also, e.g., WO2015/153513 or WO2015/138920.

The CLDN18.2 binding molecules of the present invention can also be linked to a solid-phase support which is particularly useful for immunoassay or for the purification of a target antigen. Such solid-phase supports include, but are not limited to, glass, cellulose, polyacrylamide, nylon, polystyrene, polyvinyl chloride or polypropylene.

In some embodiments, the immunoconjugates are used for treating tumours. In some embodiments, the tumours are cancers.

### IV. Nucleic acid of the present invention and host cell comprising same

In one aspect, the present invention provides a nucleic acid encoding any of the above CLDN18.2 binding molecules or the fragments thereof or any one of the chains thereof. In one embodiment, provided is a vector containing the nucleic acid. In one embodiment, the vector is an expression vector. In one embodiment, provided is a host cell containing the nucleic acid or the vector. In one embodiment, the host cell is a eukaryotic cell. In another embodiment, the host cell is selected from a yeast cell, a mammalian cell (such as CHO cell or 293 cell), or other cells suitable for the preparation of antibodies or antigen-binding fragments thereof. In another embodiment, the host cell is a prokaryotic cell.

For example, the nucleic acid of the present invention comprises a nucleic acid encoding any one of amino acid sequences as shown in SEQ ID NOs: 4, 5, 8, and 9, or a nucleic acid encoding an amino acid sequence having at least 85%, 90%, 91%, 92%, 93%, 94%, 95%, 96%, 97%, 98% or 99% identity to any one of amino acid sequences as shown in SEQ ID NOs: 4, 5, 8, and 9.

The present invention also encompasses nucleic acids that hybridize under stringent conditions with the following nucleic acids, or nucleic acids that encode a polypeptide sequence having one or more amino acid substitutions (such as conservative substitutions), deletions or insertions as compared to the following nucleic acids: a nucleic acid comprising a nucleic acid sequence encoding any one of amino acid sequences as shown in SEQ ID NOs: 4, 5, 8, and 9; or a nucleic acid comprising a nucleic acid sequence encoding an amino acid sequence having at least 85%, 90%, 91%, 92%, 93%, 94%, 95%, 96%, 97%, 98% or 99% identity to any one of amino acid sequences as shown in SEQ ID NOs: 4, 5, 8, and 9.

In one embodiment, provided is one or more vectors comprising the nucleic acid. In one embodiment, the vector is an expression vector, for example, a eukaryotic expression vector. Vectors include, but are not limited to, viruses, plasmids, cosmids, λ phages or yeast artificial chromosomes (YAC). In one embodiment, the vector is a pcDNA3.3-TOPO vector.

Once an expression vector or a DNA sequence has been prepared for expression, the expression vector can be transfected or introduced into a suitable host cell. Various techniques, such as protoplast fusion, calcium phosphate precipitation, electroporation, retroviral transduction, viral transfection, gene gun, lipid-based transfection or other conventional techniques, can be used to achieve this purpose. In the case of protoplast fusion, cells are grown in culture medium and screened for appropriate activity. Methods and conditions for culturing the resulting transfected cells and for recovering the resulting antibody molecules are known to a person skilled in the art and can be varied or optimized on the basis of the present description and methods known in the prior art, depending on the particular expression vector and mammalian host cell used.

Additionally, cells that have stably incorporated DNA into their chromosomes can be selected by introducing one or more markers that allow selection of transfected host cells. Markers may, for example, provide prototrophy, biocidal resistance (such as antibiotics) or resistance to heavy metals (such as copper) to auxotrophic hosts. A selectable marker gene can be directly linked to a DNA sequence to be expressed or introduced into the same cell by co-transformation. Additional elements may also be required for optimally synthesis of mRNA. These elements may include splicing signals, as well as transcription promoters, enhancers and termination signals.

In one embodiment, provided is a host cell containing the polynucleotide of the present invention. In some embodiments, provided is a host cell containing the expression vector of the present invention. In some embodiments, the host cell is selected from a yeast cell, a mammalian cell or other cells suitable for the preparation of antibodies. Suitable host cells include prokaryotic microorganisms, such as E. coli. Host cells can also be eukaryotic microorganisms such as filamentous fungi or yeasts, or various eukaryotic cells such as insect cells. Vertebrate cells can also be used as hosts. For example, mammalian cell lines engineered to be adapted to grow in suspension can be used. Examples of useful mammalian host cell lines include monkey kidney CV1 line transformed by SV40 (COS-7); and human embryonic kidney lines (HEK293 or 293F cells), 293 cells, baby hamster kidney cells (BHK), monkey kidney cells (CV1), African green monkey kidney cells (VERO-76), human cervical cancer cells (HELA), canine kidney cells (MDCK), Buffalo rat liver cells (BRL 3A), human lung cells (W138), human liver cells (HepG2), Chinese hamster ovary cells (CHO cells), CHO-S cells, NSO cells, and myeloma cell lines such as Y0, NS0, P3X63 and Sp2/0. For a review of mammalian host cell lines suitable for protein production, see, for example, Yazaki and Wu, Methods in Molecular Biology, Vol. 248 (ed. B.K.C. Lo, Humana Press, Totowa, NJ), pp. 255-268 (2003). In a preferred embodiment, the host cell is a CHO cell or an HEK293 cell.

### V. Production and purification of the CLDN18.2 binding molecules of the invention

In one embodiment, the present invention provides a method for preparing CLDN18.2 binding molecules, wherein the method comprises culturing a host cell containing a nucleic acid encoding the CLDN18.2 binding molecules or an expression vector containing the nucleic acid under conditions suitable for the expression of the nucleic acid encoding the CLDN18.2 binding molecules, and optionally isolating the CLDN18.2 binding molecules. In a certain embodiment, the method further comprises recovering the CLDN18.2 binding molecules from the host cell (or a host cell culture medium).

For recombinant production of the CLDN18.2 binding molecules of the present invention, a nucleic acid encoding the CLDN18.2 binding molecules of the present invention is first isolated and inserted into a vector for further cloning and/or expression in a host cell. Such nucleic acids are readily isolated and sequenced using a conventional procedure, e.g., using an oligonucleotide probe that is capable of specifically binding to a nucleic acid encoding the CLDN18.2 binding molecules of the present invention.

The CLDN18.2 binding molecules of the present invention prepared as described herein can be purified by techniques known in the prior art, such as high performance liquid chromatography, ion exchange chromatography, gel electrophoresis, affinity chromatography and steric exclusion chromatography. The actual conditions used to purify a particular protein also depend on factors such as net charge, hydrophobicity and hydrophilicity, which will be apparent to a person skilled in the art. The purity of the CLDN18.2 binding molecules of the present invention can be determined by any of a variety of well-known analytical methods, including steric exclusion chromatography, gel electrophoresis, high performance liquid chromatography, etc.

### VI. Activity assay of thee CLDN18.2 binding molecules of the present invention

The CLDN18.2 binding molecules provided herein can be identified, screened or characterized for its physical/chemical properties and/or biological activities by a variety of assays known in the art. In one aspect the CLDN18.2 binding molecules of the present invention is tested for its binding activity to an antigen, for example, by known methods such as FACS, ELISA or Western blotting. The binding to CLDN18.2 can be determined using methods known in the art, and exemplary methods are disclosed herein. In some embodiments, FACS is used to determine the binding of the CLDN18.2 binding molecules of the present invention to cell surface CLDN18.2 (e.g., human CLDN18.2).

The present invention also provides an assay for identifying CLDN18.2 binding molecules having biological activities. Biological activities may include, for example, ADCC effects, CDC effects, and the like.

Cells for use in any of the above in vitro assays include cell lines that naturally expressing CLDN18.2 or are engineered to express CLDN18.2. The cell line engineered to express CLDN18.2 is a cell line that normally does not express CLDN18.2, but expresses CLDN18.2 upon transfection of DNA encoding CLDN18.2 into the cell.

It can be understood that any of the above assays can be performed by replacing an CLDN18.2 binding molecule with the immunoconjugate of the present invention.

### VII. Pharmaceutical composition and pharmaceutical preparation

In some embodiments, the present invention provides a composition comprising any of the CLDN18.2 binding molecules or immunoconjugates thereof described herein, preferably the composition is a pharmaceutical composition. In one embodiment, the composition further comprises a pharmaceutical adjuvant material. In one embodiment, the composition (such as a pharmaceutical composition) comprises a combination of the CLDN18.2 binding molecule or the immunoconjugate thereof of the present invention, and one or more other therapeutic agents, such as chemotherapeutic agents, cytotoxic agents, other antibodies, small molecule drugs or immunomodulators, for example, anti-PD-1 antibodies or anti-PD-L1 antibodies.

In some embodiments, the composition is used for treating tumours. In some embodiments, the tumours are cancers.

The present invention also includes a composition (including a pharmaceutical composition or a pharmaceutical preparation) comprising a CLDN18.2 binding molecule or an immunoconjugate thereof and/or a composition (including a pharmaceutical composition or a pharmaceutical preparation) comprising a polynucleotide encoding a CLDN18.2 binding molecule. These compositions may also comprise suitable pharmaceutical adjuvant materials, such as pharmaceutical carriers and pharmaceutical excipients known in the art, including buffers.

As used herein, the "pharmaceutical carrier" includes any and all solvents, dispersion media, isotonic agents, absorption delaying agents, etc. that are physiologically compatible. Pharmaceutical carriers suitable for use in the present invention can be sterile liquids, such as water and oils, including those of petroleum, animal, vegetable or synthetic origin, such as peanut oil, soybean oil, mineral oil and sesame oil. Water is a preferred carrier when the pharmaceutical composition is administered intravenously. Saline solutions, aqueous dextrose and glycerol solutions can also be used as liquid carriers, particularly for injectable solutions. Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, etc. For using excipients and the use of excipients, reference can also be made to "Handbook of Pharmaceutical Excipients", fifth edition, R.C.Rowe, P.J.Seskey and S.C.Owen, Pharmaceutical Press, London, Chicago. The composition, if desired, can also contain small amounts of wetting agents or emulsifying agents, or pH buffers. These compositions can be in the form of solutions, suspensions, emulsions, tablets, pills, capsules, powders, sustained-release preparations, etc.

The pharmaceutical preparation comprising the CLDN18.2 binding molecules described herein can be prepared by mixing the CLDN18.2 binding molecules of the present invention having the desired purity with one or more optional pharmaceutical adjuvant materials (Remington's Pharmaceutical Sciences, 16th edition, Osol, A. ed. (1980)), preferably in the form of a lyophilized preparation or an aqueous solution.

The pharmaceutical compositions or preparation of the present invention may also contain more than one active ingredient, which is required for the particular indication being treated, preferably those having complementary activities that do not adversely affect each other. For example, it is desirable to also provide other anti-cancer active ingredients, such as chemotherapeutic agents, cytotoxic agents, other antibodies, small molecule drugs or immunomodulators, such as anti-PD-1 antibodies and anti-PD-L1 antibodies. Such active ingredients are suitably present in combination in an amount effective for the intended use.

Sustained-release preparations can be prepared. Suitable examples of sustained-release preparations include semipermeable matrices of solid hydrophobic polymers containing the CLDN18.2 binding molecules of the present invention, which matrices are in the form of shaped articles such as films or microcapsules.

### VIII. Combined products or kits

In some embodiments, the present invention also provides a combined product comprising the CLDN18.2 binding molecules of the present invention, or antigen-binding fragments thereof, or immunoconjugates thereof and one or more other therapeutic agents (such as chemotherapeutic agents, other antibodies, cytotoxic agents, small molecule drugs or immunomodulators). In some embodiments, other antibodies are, for example, anti-PD-1 antibodies and anti-PD-L1 antibodies.

In some embodiments, the combined product is used for treating tumours. In some embodiments, the tumours are cancers, etc.

In some regimens, two or more ingredients of the combined product may be sequentially, separately or simultaneously administered in combination to a subject.

In some embodiments, the present invention also provides a kit comprising the CLDN18.2 binding molecule, pharmaceutical composition, immunoconjugate or combined product of the present invention, and optionally a package insert directing administration.

In some embodiments, the present invention also provides a pharmaceutical product comprising the CLDN18.2 binding molecule, pharmaceutical composition, immunoconjugate or combined product of the present invention, optionally further comprising a package insert directing administration.

### IX. Use of the CLDN18.2 binding molecules of the present invention

In one aspect, the present invention relates to a method for treating a disease associated with CLDN18.2 in a subject, the method comprising administering to the subject a therapeutically effective amount of a CLDN18.2 binding molecule disclosed herein, or a pharmaceutical composition, immunoconjugate or combined product comprising same.

In some embodiments, the present invention relates to a method for treating a cancer that expresses or overexpresses CLDN18.2 in a subject, comprising administering to the subject a therapeutically effective amount of the CLDN18.2 binding molecule disclosed herein, or a pharmaceutical composition or an immunoconjugate or a combined product containing the same. In some embodiments, the cancer that expresses or overexpresses CLDN18.2 is, for example, bone cancer, blood cancer, lung cancer, hepatic cancer, pancreatic cancer, esophagus cancer, skin cancer, head and neck cancer, cutaneous or intraocular melanoma, uterine cancer, ovarian cancer, rectal cancer, cancer of the anal region, gastric cancer, colon cancer, breast cancer, prostate cancer, uterine cancer, cancers of sexual organs and reproductive organs, Hodgkin's disease, esophageal cancer, small intestine cancer, cancers of endocrine system, thyroid cancer, parathyroid carcinoma, adrenal cancer, soft tissue sarcomas, bladder cancer, renal cancer, renal cell carcinoma, renal pelvis cancer, central nervous system (CNS) tumour, neuroectodermal cancer, spinal axis tumour, glioma, meningioma, and pituitary adenoma, preferably, the cancer is gastric cancer, pancreatic cancer, esophageal cancer, ovarian cancer or lung cancer.

The subject may be a mammal, for example, a primate, preferably a higher primate, for example, human (such as a patient suffering from or at risk of suffering from the disease described herein). In one embodiment, the subject suffers from or is at risk of suffering from the disease described herein (such as the tumour described herein). In certain embodiments, the subject receives or has received other treatments such as chemotherapy treatment and/or radiation therapy.

In some embodiments, the cancers, as described herein include but are not limited to solid tumours, blood cancers, soft tissue tumours and metastatic lesions.

In some embodiments, the treatment method described herein further comprises administering to a subject or individual the CLDN18.2 binding molecule, pharmaceutical composition, immunoconjugate or combined product disclosed herein in combination with one or more other therapies, such as therapeutic methods and/or other therapeutic agents.

In some embodiments, the therapeutic methods include surgery (such as tumour resection), radiation therapy (e.g., an external beam therapy, which involves three-dimensional conformal radiation therapy in which an area of irradiation is designed), localized irradiation (e.g., irradiation directed at a preselected target or organ) or focused irradiation), *etc.* The focused irradiation may be selected from stereotactic radiosurgery, fractionated stereotactic radiosurgery and intensity modulated radiotherapy. The focused irradiation may have a radiation source selected from a particle beam (proton), cobalt-60 (photon) and a linear accelerator (X-ray), for example, as described in WO2012/177624.

Radiation therapy can be administered by one or a combination of several methods including, but not limited to, external beam therapy, internal radiation therapy, implant irradiation, stereotactic radiosurgery, systemic radiation therapy, radiotherapy and permanent or transient interstitial brachytherapy.

In some embodiments, the therapeutic agent is selected from a chemotherapeutic agent, a cytotoxic agent, other antibodies, a small molecule drug or an immunomodulator (e.g., an activator of a costimulatory molecule or an inhibitor of an immune checkpoint molecule).

Exemplary other antibodies include, but are not limited to, inhibitors of immune checkpoint molecules (e.g., anti-PD-1, anti-PD-L1, anti-TIM-3, anti-CEACAM or anti-LAG-3 antibodies), and antibodies that stimulate immune cells (e.g., agonistic anti-GITR antibodies or anti-CD 137 antibodies). Preferably, the other antibodies are selected from anti-PD-1 antibodies and/or anti-PD-L1 antibodies. More preferably, the anti-PD-1 antibody is Nivolumab from Bristol-Myers Squibb Company (BMS) and Pembrolizumab from Merck; and the anti-PD-L1 antibody is atezolizumab developed by Roche, avelumab developed cooperatively by Merck KGaA and Pfizer, and durvalumab developed by AstraZeneca.

In some embodiments, the immunomodulator is an activator or agonist of a costimulatory molecule. In one embodiment, the agonist of the costimulatory molecule is selected from an agonist (e.g., an agonistic antibody or an antigen-binding fragment thereof, or a soluble fusion) of the following molecules: OX40, CD2, CD27, CD28, CDS, ICAM-1, LFA-1 (CD11a/CD18), ICOS (CD278), 4-1BB (CD137), GITR, CD30, CD40, BAFFR, HVEM, CD7, LIGHT, NKG2C , SLAMF7, NKp80, CD160, B7-H3 or CD83 ligand.

Combination therapies of the present invention encompasses combined administration (in which two or more therapeutic agents are contained in the same preparation or separate preparations) and separate administration. In the case of separate administration, the administration of the CLDN18.2 binding molecules or immunoconjugates of the present invention, *etc.* may be performed prior to, simultaneously with and/or after administration of other therapies.

In one embodiment, the administration of the CLDN18.2 binding molecules and the administration of other therapies (e.g., therapeutic methods or therapeutic agents) occur within about one month, within about one, two or three weeks, or within about 1, 2, 3, 4, 5, or 6 days of each other.

The CLDN18.2 binding molecules (and pharmaceutical compositions or immunoconjugates comprising same) of the present invention can be administered by any suitable method, including parenteral administration, intrapulmonary administration and intranasal administration, and, if topical treatment is desired, intralesional administration. Parenteral infusion includes intramuscular, intravenous, intraarterial, intraperitoneal or subcutaneous administration. Administration can be performed by any suitable route, for example, by injection, such as intravenous or subcutaneous injection, depending to a certain extent on whether dosing is short-term or long-term. A variety of dosing schedules are encompassed herein, including but not limited to single administration, multiple administrations at multiple time points, bolus administration and pulse infusion.

To prevent or treat diseases, suitable doses of the CLDN18.2 binding molecules of the present invention (when used alone or in combination with one or more other therapeutic agents) will depend on the type of a disease to be treated, the type of the CLDN18.2 binding molecules, the severity and progression of the disease, whether the CLDN18.2 binding molecules are administered for prophylactic or therapeutic purposes, previous treatment, the clinical history and response to the CLDN18.2 binding molecules of a patient, and the judgment of the attending physician. The CLDN18.2 binding molecules are suitably administered to a patient in one treatment or a series of treatments. The dose and treatment regimen of the CLDN18.2 binding molecules can be determined by the skilled person.

It can be understood that any of the above preventions or treatments can be performed by replacing the CLDN18.2 binding molecules with the immunoconjugates, compositions or combined products of the present invention.

### X. Methods and compositions for diagnosis and detection

In certain embodiments, any of the CLDN18.2 binding molecules provided herein can be used to detect the presence of CLDN18.2 in a biological sample. As used herein, the term "detect" includes quantitative or qualitative detection. Exemplary detection methods may involve immunohistochemistry, immunocytochemistry, flow cytometry (e.g., FACS), magnetic beads complexed with antibody molecules and ELISA. In certain embodiments, the biological sample is blood, blood serum, or other body fluid samples of biological origin. In certain embodiments, the biological sample comprises a cell or a tissue. In some embodiments, the biological sample is from a hyperproliferative or cancerous lesion.

In one embodiment, provided are CLDN18.2 binding molecules for use in a diagnosis or detection method. In another aspect, provided is a method for detecting the presence of CLDN18.2 in a biological sample. In certain embodiments, the method comprises detecting the presence of CLDN18.2 protein in a biological sample. In certain embodiments, CLDN18.2 is human CLDN18.2. In certain embodiments, the method comprises contacting the biological sample with the CLDN18.2 binding molecules as described herein under conditions that allow the CLDN18.2 binding molecules to bind to CLDN18.2, and detecting whether a complex is formed between the CLDN18.2 binding molecules and CLDN18.2. The formation of the complex indicates the presence of CLDN18.2. The method may be an in vitro or in vivo method. In one embodiment, the CLDN18.2 binding molecules are used to select a subject suitable for being treated with the CLDN18.2 binding molecules, e.g., wherein the CLDN18.2 is a biomarker for selecting the subject.

In one embodiment, the CLDN18.2 binding molecules of the present invention can be used to diagnose cancers or tumours, for example, to evaluate (e.g., monitor) the treatment or progression, diagnosis and/or staging of the disease described herein (e.g., a hyperproliferative or cancerous disease) in a subject. In certain embodiments, labelled CLDN18.2 binding molecules are provided. Labels include, but are not limited to, labels or moieties that are detected directly (such as fluorescent labels, chromophore labels, electron-dense labels, chemiluminescent labels and radioactive labels), and moieties that are detected indirectly (such as enzymes or ligands), for example, by an enzymatic reaction or a molecular interaction. Exemplary labels include, but are not limited to, radioisotopes ³²P, ¹⁴C, ¹²⁵I, ³H and ¹³¹I, fluorophores such as rare earth chelates or luciferin and derivatives thereof, rhodamine and derivatives thereof, dansyl, umbelliferone, luciferase, such as firefly luciferase and bacterial luciferase (U.S. Pat. No. 4,737,456), fluorescein, 2,3-dihydrophthalazinedione, horseradish peroxidase (HR), alkaline phosphatase, β-galactosidase, glucoamylase, lysozyme, carbohydrate oxidase (such as glucose oxidase, galactose oxidase and glucose-6-phosphate dehydrogenase), heterocyclic oxidase (such as uricase and xanthine oxidase), enzymes that oxidize dye precursors with hydrogen peroxide (such as HR, lactoperoxidase or microperoxidase), biotin/avidin, spin labels, phage labels, stable free radicals, *etc.*

In some embodiments of any of the inventions provided herein, the sample is obtained prior to treatment with the CLDN18.2 binding molecules. In some embodiments, the sample is obtained after the cancer has metastasized. In some embodiments, the sample is formalin fixed paraffin embedded (FFPE). In some embodiments, the sample is a biopsy (e.g., a core biopsy), a surgical specimen (e.g., a specimen from a surgical resection) or a fine needle aspirate.

In some embodiments, the CLDN18.2 is detected prior to treatment, e.g., prior to initiation of treatment or prior to certain treatment after a treatment interval.

In some embodiments, provided is a method for treating tumours, comprising: detecting the presence of CLDN18.2 in a subject (e.g., a sample) (e.g., a subject sample containing cancer cells), thereby determining a value of CLDN18.2; comparing the value of CLDN18.2 with a control value (e.g., a value of CLDN18.2 in a sample of a healthy individual); and if the value of CLDN18.2 is greater than the control value, administering to the subject a therapeutically effective amount of CLDN18.2 binding molecules (e.g., the CLDN18.2 binding molecules described herein), optionally in combination with one or more other therapies, thereby treating tumours.

It can be understood that various embodiments described in various sections of the present invention, such as diseases, therapeutic agents, therapeutic methods and administration, are equally applicable to, or may be combined with, embodiments of other sections of the present invention. Embodiments described in various sections of the present invention, such as properties, uses and methods, applicable to CLDN18.2 binding molecules are equally applicable to compositions, conjugates, combined products, kits, etc. comprising the CLDN18.2 binding molecules.

### Examples

The following examples are intended to illustrate the present invention only and therefore should not be construed as limiting the present invention in any way.

### Example 1 Construction and identification of overexpressing cell lines

### 1.1 Construction and identification of a NUGC4 cell line overexpressing human CLDN18.2

The gastric cancer cell line NUGC4 cell line overexpressing human CLDN18.2 (hereinafter referred to as hCLDN18.2-NUGC4) was constructed by lentiviral transfection and identified by the antibody IMAB362 (an antibody specifically binding to CLDN18. 2, Ganymed, Germany).

The specific method was as follows: 5 × 10⁴ human gastric cancer cells (NUGC4 cells, obtained from BNCC strain library, No. BNCC341962) in good state were taken; the packaged lentivirus containing the human CLDN18.2 sequence (SEQ ID NO: 10) was added at a multiplicity of infection (MOI) of 30 : 1 (with reference to the lentivirus packaging method in example 3 of CN 109485734 B), and mixed thoroughly; then an IMDM complete medium (Gibico, 2192731) containing 5 µg/mL polybrene (Polybrene, OBiO Technology (Shanghai) Corp., Ltd.) was added, mixed evenly, and incubated for 20 hours in a constant temperature incubator at 37°C and 5% CO₂; the medium was then removed and replaced with a fresh IMDM complete medium, and incubation was continued for 24 hours; next, lentivirus-transfected NUGC4 cells were seeded into a 96-well plate at an average cell density of 0.5 cells/well, and puromycin was added at a final concentration of 2 µg/mL for resistance pressure screening; incubation was performed for 2-3 weeks in a constant temperature incubator at 37°C and 5% CO₂, and clones were picked and identified using the antibody IMAB362; and finally, a NUGC4 cell line overexpressing human CLDN18.2 was successfully obtained, also called "hCLDN18.2-NUGC4 cell line" herein.

### 1.2 Construction and identification of an HEK293 cell line overexpressing human CLDN18.2

The DNA sequence of full-length human CLDN18.2 (SEQ ID NO: 10) was constructed into pLVX-puro plasmid (Clontech, Cat#632164). Then, the resulting plasmids were transformed into HEK293 cells (ATCC^{®}CRL-1573^{™}) via electrotransformation. Through screening, cell culture was carried out with reference to example 1.1, resistance pressure screening was performed with puromycin, and the clones were identified using the antibody IMAB362; finally, a HEK293 cell line overexpressing human CLDN18.2 was successfully obtained, also called "hCLDN18.2-HEK293 cell line" herein.

### 1.3 Construction and identification of a CD16a(F158)-NF-AT-Jurkat cell line

Firstly, the NF-AT-Jurkat cell line was constructed; the pGL4.30 plasmid (Promega, catalogue number: E8481) containing the NFAT response element (NFAT-RE) DNA sequence was electroporated into Jurkat cells (ATCC^{®}TIB-152) by an electroporator (Invitrogen, Neon^{™} Transfection System, MP922947). After electrotransformation, hygromycin B (Shanghai BasalMedia Technologies Co., LTD., S160J7) was used at a final concentration of 500 µg/mL for resistance pressure screening; the clone growth of cell lines was observed in about 2-3 weeks, and the cell lines that form clones were picked for identification. The identification method was as follows: some clones were transferred to a 96-well white bottom plate (Corning, 3610), stimulated with PMA (10 ng/mL) and ionomycin (1 nM), and incubated in an incubator at 37°C and 5% CO₂ for 6 hours, and then Bright glo (Vazyme, DD1204-01) was added; after reading the signal value with a microplate reader (Molecular Devices: Spectramax i3x), the expression levels of NF-κB in different clones were evaluated to obtain a Jurkat cell line that highly expresses the NF-AT gene (referred to as NF-AT-Jurkat cell line).

The NF-AT-Jurkat cell line was taken; the packaged lentivirus containing CD16a (F158) sequence (UniProtKB - P08637, amino acid at position 158 was F phenylalanine) was added at a multiplicity of infection (MOI) of 20 : 1, and puromycin was added at a final concentration of 2 µg/mL for resistance pressure screening; incubation was performed for 2-3 weeks in a constant temperature incubator at 37°C and 5% CO₂; the clones were picked for identification (with reference to example 7 of the present application for the identification method); and finally, the CD16a(F158)-NF-AT-Jurkat cell line was successfully obtained.

### Example 2 Affinity maturation engineering of anti-CLDN18.2 nanobody

In order to improve the specific binding of Nanobody Nb-NA3S-H1 to human CLDN18.2, this example carried out affinity maturation engineering of nanobody Nb-NA3S-H1.

For each of the three CDRs defined by AbM in the nanobody Nb-NA3S-H1, a single site or two consecutive sites were mutated, an affinity matured phage display library was constructed, and the affinity matured molecules were screened using phage display technology, and the screening method refers to example 3 in WO2020238730 A1.

Through affinity maturation engineering, the candidate nanobody Nb-NA3S-H1-T4 was obtained. Using AbM to define CDRs, the complementarity determining region sequence of the candidate nanobody Nb-NA3S-H1-T4 was determined. The amino acid sequences of the CDRs are shown in Table 2.

**Table 2 CDR sequences of parent antibody Nb-NA3S-H1 and affinity matured antibody Nb-NA3S-H1-T4**

| Antibody name | CDR1 | CDR2 | CDR3 |
|---|---|---|---|
| Nb-NA3S-H1-T4 | GSIFHIPVMG (SEQ ID NO: 1) | GISRGGTTN (SEQ ID NO: 2) | LVVSGIGSTLEV (SEQ ID NO: 3) |
| Nb-NA3S-H1 | GSIFNIPVMG (SEQ ID NO: 11) | GISTGGTTN (SEQ ID NO: 12) | LVVSGIGSTLEV (SEQ ID NO: 3) |

### Example 3 Humanization of affinity matured nanobody Nb-NA3S-H1-T4

The variable region sequence of nanobody Nb-NA3S-H1-T4 was aligned with the human antibody germline gene (Germline) database and 1-3 germline genes that had high homology with nanobody Nb-NA3S-H1-T4 were found, while taking into account the druggability of germline genes, appropriate germline gene Germline templates were selected for alignment.

Homology modelling was performed on the nanobody Nb-NA3S-H1-T4. The homology modelling referred to the nanobody structure model of the PDB database (http://www.rcsb.org/). Combined with the structural model of the nanobody Nb-NA3S-H1-T4 and the situation of non-human sites, a combinatorial backmutation design was carried out. The backmutation design avoided the introduction of potential post-translational modification sites, and finally, the candidate nanobody Nb-NA3S-H1-T4-hVH6 was designed with a humanization degree of 96.67%.

The amino acid sequences (heavy chain single domain variable regions) of candidate nanobodies Nb-NA3S-H1-T4 and Nb-NA3S-H1-T4-hVH6 are shown in Table 3.

**Table 3 Amino acid sequences of anti-CLDN18.2 nanobodies**

| Antibody name | Amino acid sequence |
|---|---|
| Nb-NA3S-H1-T4 | QVQLVESGGGLVQPGGSLRLSCAASGSIFHIPVMGWYRQAPGKQ RELVAGISRGGTTNYGDSVKGRFTISRDNAKNTVYLQMNSLKPE DTAVYYCNVLVVSGIGSTLEVWGQGTLVTVSS(SEQ ID NO: 4) |
| Nb-NA3S-H1-T4-hVH6 | QVQLVESGGGLVQPGGSLRLSCAASGSIFHIPVMGWYRQAPGKG LELVAGISRGGTTNYADSVKGRFTISRDNAKNTLYLQMNSLRAE DTAVYYCNVLVVSGIGSTLEVWGQGTLVTVSS (SEQ ID NO: 5) |

### Example 4 Construction of anti-CLDN18.2 heavy-chain antibodies

The C-terminuses of the amino acid sequences of nanobody Nb-NA3S-H1, nanobody Nb-NA3S-H1-T4, and nanobody Nb-NA3S-H1-T4-hVH6 were respectively connected to the N-terminus of the Fc region of human IgG1 (the amino acid sequence was shown in SEQ ID NO: 6), and anti-CLDN18.2 heavy-chain antibodies NA3SH1, NA3SH1-T4 and NA3SH1-T4-hVH6 were constructed.

Specifically, the gene sequences of nanobody Nb-NA3S-H1, nanobody Nb-NA3S-H1-T4, nanobody Nb-NA3S-H1-T4-hVH6, and hIgG1 Fc region (amino acid sequence as shown in SEQ ID NO: 6) were obtained by PCR amplification, respectively; the gene sequence of each nanobody was connected to the gene sequence of the hIgG1 Fc region by overlap extension PCR and then constructed into the modified eukaryotic expression vector plasmid pcDNA3.3-TOPO (Invitrogen, Cat. No.: K830001) through homologous recombination; the expression vector comprising the gene sequence of each anti-CLDN18.2 heavy-chain antibody was transformed into *E. coli* SS320 cells and cultured at 37°C overnight. An endotoxin-free plasmid extraction kit (OMEGA, D6950-01) was used for plasmid extraction to obtain an endotoxin-free anti-CLDN18.2 heavy-chain antibody plasmid for eukaryotic expression.

The corresponding amino acid sequences of anti-CLDN18.2 heavy-chain antibodies are provided in Table 4.

**Table 4 Amino acid sequences of anti-CLDN18.2 heavy-chain antibodies**

| Heavy-chain antibody name | Amino acid sequence |
|---|---|
| NA3SH1 | SEQ ID NO: 7 |
| NA3SH1-T4 | SEQ ID NO: 8 |
| NA3SH1-T4-hVH6 | SEQ ID NO: 9 |

### Example 5 Expression, purification and analysis of physicochemical properties of anti-CLDN18.2 heavy-chain antibodies

### 5.1 Expression and purification of anti-CLDN18.2 heavy-chain antibodies

The anti-CLDN18.2 heavy-chain antibodies were expressed using the ExpiCHO transient expression system (Thermo Fisher, A29133). The specific method was as follows: On the day of transfection, the ExpiCHO cell density should be about 7 × 10⁶ to 1 × 10⁷ viable cells/mL, and the cell survival rate should be > 98%. The cells were adjusted to a final concentration of 6 × 10⁶ cells/mL with fresh ExpiCHO expression medium pre-warmed at 37°C. The plasmids of interest were diluted with OptiPRO^{™} SFM pre-chilled at 4°C to make a plasmid dilution (1 µg of the anti-CLDN18.2 heavy-chain antibody plasmids prepared in example 4 were added to 1 mL of the culture medium); meanwhile, ExpiFectamine^{™}CHO was diluted with OptiPRO^{™}SFM to make an ExpiFectamine^{™}CHO dilution; and the plasmid dilution and the ExpiFectamine^{™}CHO dilution were mixed in equal volumes and mixed uniformly by gently pipetting to prepare an ExpiFectamine^{™}CHO/plasmid DNA mixture, and incubated at room temperature for 1-5 minutes. The prepared ExpiCHO cell suspension was slowly added with gentle shaking, placed on a cell culture shaker and cultured at 37°C and 8% CO₂. 18-22 h after transfection, ExpiCHO^{™}Enhancer and ExpiCHO^{™}Feed were added to the culture liquid, and the shake flask was placed in a 32°C shaker under 5% CO₂ for further culture. On day 5 after transfection, ExpiCHO^{™}Feed with the same volume was added slowly, and meanwhile the cell suspension was mixed gently and uniformly. 7-15 days after transfection, the cell culture supernatant expressing the protein of interest was centrifuged at 15000 g for 10 min. The resulting supernatant was affinity purified with MabSelect SuRe LX (GE, 17547403), and then the protein of interest was eluted with 100 mM sodium acetate (pH 3.0), followed by neutralization with 1 M Tris-HCl, and finally the resulting protein was exchanged into PBS buffer through an ultrafiltration concentration tube (Millipore, UFC901096).

### 5.2 Identification of anti-CLDN18.2 heavy-chain antibodies by SDS-PAGE

Preparation of non-reducing solution: 1 µg of each heavy-chain antibody and the reference product Ipilimumab (also abbreviated as IPI, prepared by a method similar to that in example 5.1) was added to 5×SDS loading buffer and 40 mM iodoacetamide, heated under a dry bath at 75°C for 10 min, cooled to room temperature, and centrifuged at 12000 rpm for 5 min and the supernatant was taken.

Preparation of reducing solution: 2 µg of each heavy-chain antibody and reference IPI was added to 5×SDS loading buffer and 5 mM DTT, heated under a dry bath at 100°C for 10 min, cooled to room temperature, and centrifuged at 12000 rpm for 5 min and the supernatant was taken.

Each supernatant was added to Bis-tris 4-15% gradient gel (purchased from GenScript) for electrophoresis at a constant pressure of 110 V. When Coomassie Brilliant Blue migrated to the bottom of the gel, the running was stopped. The gel piece was removed and placed in Coomassie Brilliant Blue staining solution for 1-2 h. The staining solution was discarded, and destaining solution was added and the destaining solution was replaced 2-3 times as needed. The gel piece was destained until the gel background was transparent and then stored in deionized water. After destaining, the gel piece was scanned with an EPSON V550 colour scanner, and the purity of the reduced and non-reduced bands was calculated using ImageJ according to the peak area normalization method.

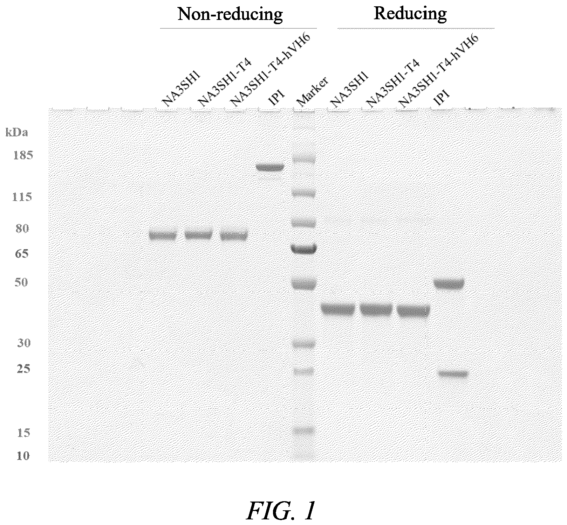

The results are shown in Fig. 1: The bands in non-reducing gels for each heavy-chain antibody and the reference IPI were in line with the expected size and the purity was above 90%.

### 5.3 Identification of monomer purity of anti-CLDN18.2 heavy-chain antibodies by SEC-HPLC

Material preparation: 1. Mobile phase: 150 mmol/L phosphate buffer, pH 7.4; 2. Sample preparation: Each anti-CLDN18.2 heavy-chain antibody was diluted to 0.5 mg/mL with mobile phase solution. The flow rate of the Agilent HPLC 1100 column (XBridge BEH SEC 3.5 µm, 7.8 mm I.D. × 30 cm, Waters) was set to 0.8 mL/min, the load volume was 20 µL, and the VWD detector wavelengths were 280 nm and 214 nm.

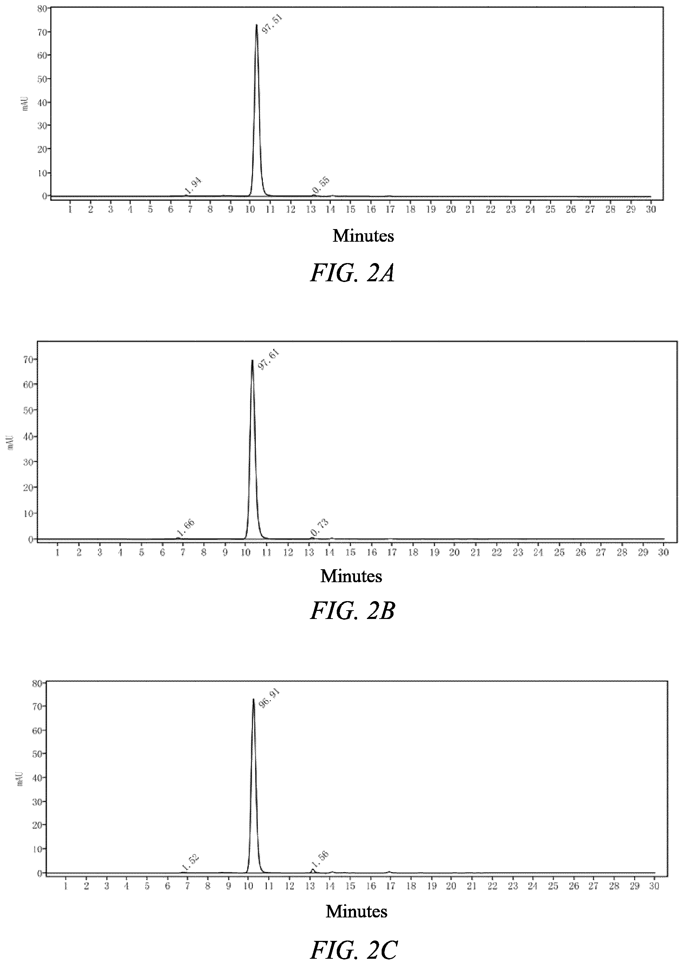

The size-exclusion high-performance liquid chromatography (SEC-HPLC) results of the anti-CLDN18.2 heavy-chain antibody in this example are as follows: the percentages of high molecular polymers, anti-CLDN18.2 heavy-chain antibody monomers and low molecular substances in the sample were calculated according to the area normalization method. The results are shown in Figs. 2A-2C and Table 5.

As can be seen from Figs. 2A-2C and Table 5, the expression level of heavy-chain antibody NA3SH1-T4-hVH6 is more than three times that of heavy-chain antibody NA3SH1, and nearly twice that of heavy-chain antibody NA3 SH1-T4; and the SEC-HPLC results show that the heavy-chain antibody NA3SH1-T4-hVH6 monomer has the highest percentage, which also means that the content of soluble aggregates and shear products in the product is the lowest.

**Table 5 Physicochemical data of anti-CLDN18.2 heavy-chain antibodies**

| Heavy-chain antibody name | Molecular weight (kDa) | Isoelectric point | Extinction coefficient | SDS-PAGE (%) | Transient expression (µg/mL) | SEC-HPLC (%) |
|---|---|---|---|---|---|---|
| NA3SH1-T4 | 80 | 8.28 | 1.37 | > 90.0 | 5230.00 | 97.51 |
| NA3SH1-T4-hVH6 | 80 | 8.29 | 1.41 | > 90.0 | 10300.00 | 97.61 |
| NA3SH1 | 80 | 8.03 | 1.37 | > 90.0 | 2910.00 | 96.91 |

### Example 6 Analysis of affinity activity of anti-CLDN18.2 heavy-chain antibodies

### 6.1 Binding ability of anti-CLDN18.2 heavy-chain antibodies to hCLDN18.2-HEK293 cells

hCLDN18.2-HEK293 cells in the exponential growth phase were collected, centrifuged at 300 g to remove the supernatant; the cells were resuspended in FACS buffer (PBS containing 1% BSA) and counted and the cell suspension density was adjusted to 2 × 10⁶ cells/mL. Subsequently, hCLDN18.2-HEK293 cells were added into a 96-well round-bottom plate at 100 µL per well, and centrifuged at 300 g to remove the supernatant. Different concentrations of heavy-chain antibody NA3SH1-T4, heavy-chain antibody NA3SH1-T4-hVH6, heavy-chain antibody NA3SH1 as a control, and human IgG1 isoform antibody as an isoform control were added to the corresponding wells. The cells were resuspended and placed at 4°C for incubation for 1 h. The incubated cell mixture was washed 3 times, PE-labelled anti-human-IgG-Fc flow cytometry antibody (Abeam, catalogue number: 98596) was add, resuspended and placed at 4°C for incubation for 30 minutes. The incubated cell mixture was washed 3 times, 200 µL of FACS buffer was added to resuspend the cells, and finally detected and analysed by a flow cytometer (Beckman, CytoFLEX AOO-1-1102). Data were analysed using PRISM^{™} (GraphPad Software, San Diego, CA), and EC₅₀ values were calculated.

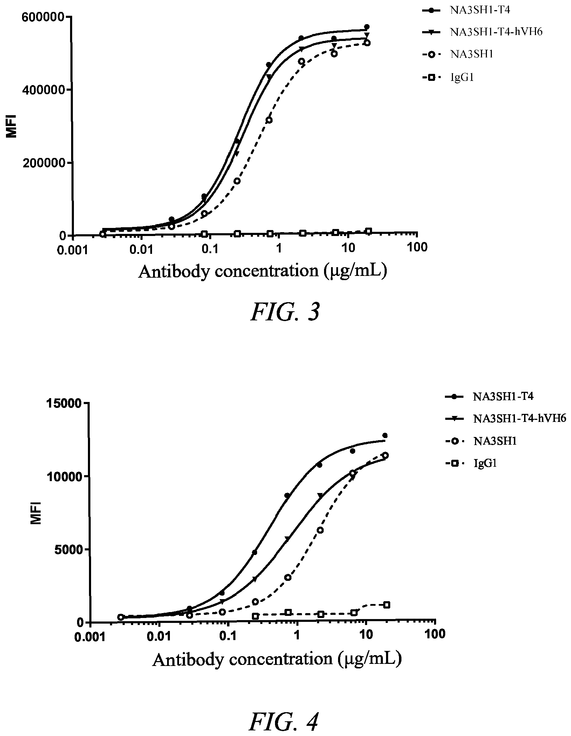

The FACS binding assay results are shown in Fig. 3. Both the heavy-chain antibody NA3SH1-T4 and the heavy-chain antibody NA3SH1-T4-hVH6 showed significantly better binding ability to CLDN18.2 than the control NA3SH1. Among them, the EC₅₀ of NA3SH1-T4 = 0.2736 µg/mL, EC₅₀ of NA3SH1-T4-hVH6 = 0.3099 µg/mL, EC₅₀ of NA3SH1 = 0.5356 µg/mL.

### 6.2 Binding ability of anti-CLDN18.2 heavy-chain antibodies to hCLDN18.2-NUGC4 cells

hCLDN18.2-NUGC4 cells in the exponential growth phase were collected, centrifuged at 300 g to remove the supernatant; the cells were resuspended in FACS buffer (PBS containing 1% BSA) and counted and the cell suspension density was adjusted to 2 × 10⁶ cells/mL. Subsequently, hCLDN18.2-NUGC4 cells were added into a 96-well round-bottom plate at 100 µL per well, and centrifuged at 300 g to remove the supernatant. Different concentrations of heavy-chain antibody NA3SH1-T4, heavy-chain antibody NA3SH1-T4-hVH6, heavy-chain antibody NA3SH1 as a control, and human IgG1 isoform antibody as an isoform control were added to the corresponding wells. The cells were resuspended and placed at 4°C for incubation for 1 h. The incubated cell mixture was washed 3 times, PE-labelled anti-human-IgG-Fc flow cytometry antibody (Abeam, catalogue number: 98596) was add, resuspended and placed at 4°C for incubation for 30 minutes. The incubated cell mixture was washed 3 times, 200 µL of FACS buffer was added to resuspend the cells, and finally detected and analysed by a flow cytometer (Beckman, CytoFLEX AOO-1-1102). Data were analysed using PRISM^{™} (GraphPad Software, San Diego, CA), and EC₅₀ values were calculated.

The FACS binding assay results are shown in Fig. 4. Both the heavy-chain antibody NA3SH1-T4 and the heavy-chain antibody NA3SH1-T4-hVH6 showed significantly better binding ability to CLDN18.2 than the heavy-chain antibody NA3SH1 as the control. Among them, the EC₅₀ of NA3SH1-T4 = 0.4047 µg/mL, EC₅₀ of NA3SH1-T4-hVH6 = 0.8465 µg/mL, EC₅₀ of NA3SH1 = 2.147 µg/mL.

### 6.3 Binding ability of anti-CLDN18.2 heavy-chain antibodies to hCLDN18.2-KATOIII cells

hCLDN18.2-KATOIII cells were made in house, and the preparation method refers to the construction of human CLDN18.2-KATOIII tumour cell line in example 1 of CN112480248 A.

Using a method similar to that described in example 6.1, hCLDN18.2-KATOIII cells were used to determine the binding ability of the anti-CLDN18.2 heavy-chain antibodies to hCLDN18.2-KATOIII cells. Data were analysed using PRISM^{™} (GraphPad Software, San Diego, CA), and EC₅₀ values were calculated.

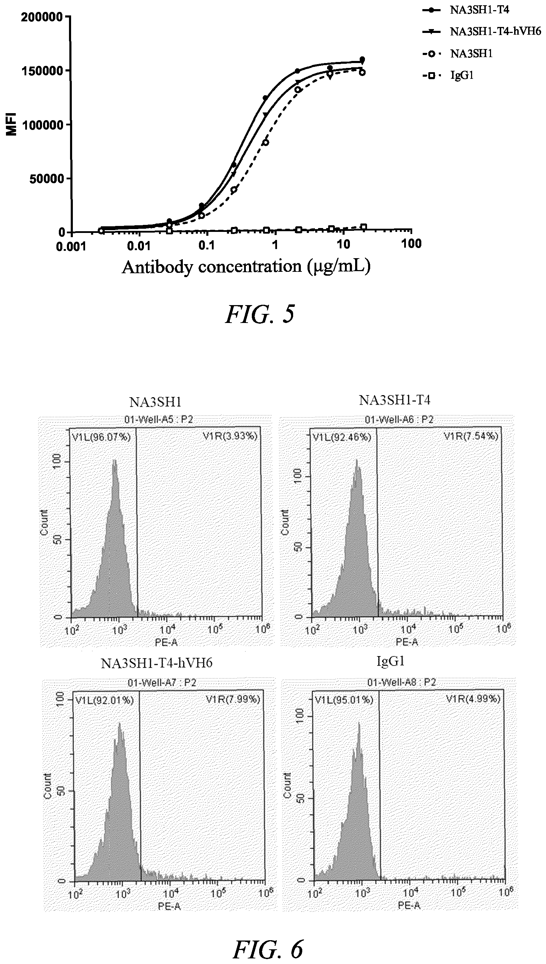

The FACS binding assay results are shown in Fig. 5. Both the heavy-chain antibody NA3SH1-T4 and the heavy-chain antibody NA3SH1-T4-hVH6 showed significantly better binding ability to CLDN18.2 than the heavy-chain antibody NA3SH1 as the control. Among them, the EC₅₀ of NA3SH1-T4 = 0.3298 µg/mL, EC₅₀ of NA3SH1-T4-hVH6 = 0.3984 µg/mL, EC₅₀ of NA3SH1 = 0.6183 µg/mL.

### 6.4 Binding ability of anti-CLDN18.2 heavy-chain antibodies to hCLDN18.1-HEK293 cells

Heavy-chain antibodies at 100 µg/mL and target cells hCLDN18.1-HEK293 (made in house, referring to 1.3.2 of example 1 of CN112480248 A for the preparation method) were incubated for 1 h at 4°C, and then rinsed three times with the above-mentioned FACS buffer, and 0.5 µg (0.5 mg/mL) of PE-labelled goat antihuman IgG Fc antibody (Abeam, catalogue number: ab98596) was added and incubated at 4°C for 30 min. rinsed three times with the FACS buffer. The cells were resuspended by adding 200 µL of FACS buffer to the cells, and finally detected by a flow cytometer (Beckman, CytoFLEX AOO-1-1102), and the binding intensity and the positive rate of cell binding were recorded.

As shown in Fig. 6, at a high concentration of 100 µg/mL, the positivity rate of binding of the heavy-chain antibody NA3SH1-T4-hVH6, the heavy-chain antibody NA3SH1-T4, and the heavy-chain antibody NA3SH1 as the control to hCLDN18.1-HEK293 cells was very close to the positivity rate of binding of human IgG 1 isoform antibodies to hCLDN18.1-HEK293 cells, indicating that neither the heavy-chain antibody NA3SH1-T4 nor the heavy-chain antibody NA3SH1-T4-hVH6 bind to hCLDN18.1 protein.

### Example 7 ADCC effect of anti-CLDN18.2 heavy-chain antibodies

For the anti-CLDN18.2 heavy-chain antibody, upon its Fc end binds to CD16a (F158) or CD16a (V158) on Jurkat cells and its VHH end binds to CLDN18.2 on the cells, NF-AT protein expression inside Jurkat cells would be activated. NF-AT binds to the NF-AT response element and triggers its downstream luciferase expression. By stimulating with anti-CLDN18.2 heavy-chain antibodies of the present invention with different concentration gradients, a fluorescence reading curve with protein concentration dependence would be obtained, thereby evaluating the ADCC activity of the antibodies.

### 7.1 ADCC effect of anti-CLDN18.2 heavy-chain antibodies on hCLDN18.2-HEK293 cells

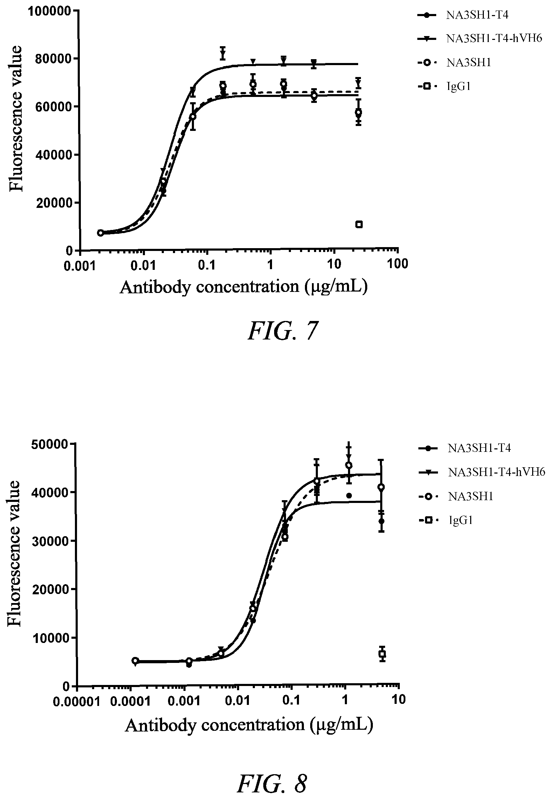

50 µL of hCLDN18.2-HEK293 cells as target cells at a density of 4 × 10⁵ cells/mL and CD16a(F158)-NF-AT-Jurkat cells as effector cells at a density of 4 × 10⁶ cells/mL was added to each well of a 96-well cell culture plate. The target cells and the effector cells were mixed at a ratio of 1 : 1 and added to a 96-well white-sided transparent bottom cell culture plate and placed in a 37°C incubator for overnight culture (16-20 hours). 50 µL of gradient diluted heavy-chain antibody NA3SH1-T4, heavy-chain antibody NA3SH1-T4-hVH6, heavy-chain antibody NA3SH1 as a control, and human IgG1 isoform antibody as an isoform control was added, respectively, and incubated in a 37°C incubator for 6 hours. 50 µL of Bright-Lite (vazyme, Cat. No. DD1204-03) was added to each well; the mixture was incubated in the dark for 10 min; and the fluorescence signal was detected. The ADCC detection results are shown in Fig. 7.

As can be seen from Fig. 7, the heavy-chain antibody NA3SH1-T4-hVH6 showed significantly stronger ADCC killing effect on hCLDN18.2-HEK293 cells than the heavy-chain antibody NA3SH1 used as the control; the heavy-chain antibody NA3SH1-T4 showed an ADCC killing effect on hCLDN18.2-HEK293 cells that was comparable to that of the heavy-chain antibody NA3SH1 as the control.

### 7.2 ADCC effect of anti-CLDN18.2 heavy-chain antibodies to hCLDN18.2-KATOIII cells

For the detection method of ADCC effect on hCLDN18.2-KATOIII cells, referring to example 7.1. The ADCC detection results are shown in Fig. 8.

As can be seen from Fig. 8, compared with the heavy-chain antibody NA3SH1 as the control and the heavy-chain antibody NA3SH1-T4, the heavy-chain antibody NA3SH1-T4-hVH6 caused the best ADCC killing effect on hCLDN18.2-KATOIII cells.

### 7.3 ADCC effect of anti-CLDN18.2 heavy-chain antibodies on hCLDN18.2-NUGC4 cells

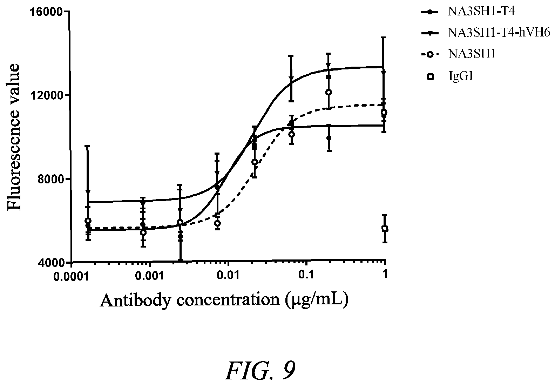

For the detection method of ADCC effect on hCLDN18.2-NUGC4 cells, referring to example 7.1. The ADCC detection results are shown in Fig. 9.

As can be seen from Fig. 9, the heavy-chain antibody NA3SH1-T4-hVH6 showed significantly stronger ADCC killing effect on hCLDN18.2-NUGC4 cells than the heavy-chain antibody NA3SH1 used as the control; the heavy-chain antibody NA3SH1-T4 showed an ADCC killing effect on hCLDN18.2-NUGC4 cells that was comparable to that of the heavy-chain antibody NA3SH1 as the control.

## Claims

1. A CLDN18.2 binding molecule, **characterized in that** the CLDN18.2 binding molecule comprises at least one single domain antibody (sdAb) portion that specifically binds to CLDN18.2, wherein the sdAb portion comprises three complementarity determining regions, namely CDR1, CDR2 and CDR3, respectively, wherein:
(a) the CDR1 comprises the amino acid sequence of SEQ ID NO: 1, or a variant with 1 or 2 amino acid changes in the amino acid sequence of SEQ ID NO: 1;
(b) the CDR2 comprises the amino acid sequence of SEQ ID NO: 2, or a variant with 1 or 2 amino acid changes in the amino acid sequence of SEQ ID NO: 2; and
(c) the CDR3 comprises the amino acid sequence of SEQ ID NO: 3, or a variant with 1 or 2 amino acid changes in the amino acid sequence of SEQ ID NO: 3,
in which the amino acid change is an amino acid addition, an amino acid deletion or a conservative amino acid substitution, preferably, the sdAb portion is a camelid VHH, a partially humanized or fully humanized VHH, or a chimeric VHH.

2. The CLDN18.2 binding molecule of claim 1, **characterized in that** the sdAb portion comprises: CDR1 comprising the amino acid sequence SEQ ID NO: 1, CDR2 comprising the amino acid sequence SEQ ID NO: 2 and CDR3 comprising the amino acid sequence SEQ ID NO: 3.

3. The CLDN18.2 binding molecule of claim 1 or 2, **characterized in that** the sdAb portion comprises
(i) the amino acid sequence of SEQ ID NO: 4 or 5; or
(ii) an amino acid sequence having at least 85%, 90%, 91%, 92%, 93%, 94%, 95%, 96%, 97%, 98% or 99% identity to the amino acid sequence of SEQ ID NO: 4 or 5.

4. The CLDN18.2 binding molecule of any one of claims 1 to 3, **characterized in that** the sdAb portion is linked at the N-terminus or C-terminus to another protein domain, for example to the Fc region of an immunoglobulin, for example to the Fc region from an IgG, such as IgG1, IgG2, IgG3 or IgG4; or, for example, the sdAb portion is linked to a fluorescent protein.

5. The CLDN18.2 binding molecule of any one of claims 1 to 4, **characterized in that** the CLDN18.2 binding molecule has one or more of the following properties:
(1) binding to CLDN18.2, such as human CLDN18.2, with high affinity, for example, the EC₅₀ of the binding between the CLDN18.2 binding molecule and CLDN18.2 on a cell surface is about 0.1 µg/mL to about 10 µg/mL, preferably, about 0.1 µg/mL to about 1 µg/mL;
(2) specifically binding to CLDN18.2 and not binding to CLDN18.1;
(3) killing CLDN18.2-positive cancer cells through antibody-dependent cell-mediated cytotoxicity and/or complement-dependent cytotoxicity.

6. The CLDN18.2 binding molecule of any one of claims 1 to 5, **characterized in that** the CLDN18.2 binding molecule is a bispecific or multispecific antibody, preferably, the bispecific antibody molecule specifically binds to the CLDN18.2 molecule and a second target protein, wherein the second target protein is selected from, for example:
(1) a tumour-specific antigen or a tumour-associated antigen, such as an epidermal growth factor receptor (EGFR1), HER2/neu, CD20, an insulin-like growth factor receptor (IGF-1R), a carcinoembryonic antigen, a prostate-specific membrane antigen (PSMA), Mucin-1, CD30, CD33, CD137, cMet, or angiopoietin-2 (Ang-2);
(2) an immune checkpoint molecule of an immune cell, for example, PD1, CTLA-4, TIM-3, or LAG-3;
(3) an immune costimulatory molecule of an immune cell, for example, OX40, ICOS, TLR2 or CD27;
(4) a cytokine, for example, IL-1, IL-2, IL-7, IL-15 or IL-33.

7. An isolated nucleic acid, **characterized in that** the isolated nucleic acid encodes the CLDN18.2 binding molecule of any one of claims 1 to 6.

8. A vector comprising the nucleic acid of claim 7, **characterized in that** preferably the vector is an expression vector, for example, a pcDNA3.3-TOPO vector.

9. A host cell comprising the nucleic acid of claim 7 or the vector of claim 8, **characterized in that** preferably the host cell is a prokaryotic cell or a eukaryotic cell, more preferably the host cell is selected from an *E. coli* cell, a yeast cell, or a mammalian cell, and most preferably the host cell is an HEK293 cell or a CHO cell.

10. A method for preparing the CLDN18.2 binding molecule of any one of claims 1 to 6, **characterized in that** the method comprises culturing the host cell of claim 9 under conditions suitable for the expression of a nucleic acid encoding the CLDN18.2 binding molecule of any one of claims 1 to 6, and optionally isolating the CLDN18.2 binding molecule, and optionally the method further comprises recovering the CLDN18.2 binding molecule from the host cell.

11. An immunoconjugate, **characterized in that** the immunoconjugate comprises the CLDN18.2 binding molecule of any one of claims 1 to 6, and other substances, such as a cytotoxic agent.

12. A pharmaceutical composition, **characterized in that** the pharmaceutical composition comprises the CLDN18.2 binding molecule of any one of claims 1 to 6 or the immunoconjugate of claim 11, and optionally a pharmaceutical adjuvant material.

13. A pharmaceutical composition, **characterized in that** the pharmaceutical composition comprises the CLDN18.2 binding molecule of any one of claims 1 to 6 or the immunoconjugate of claim 11, and other therapeutic agents, and optionally a pharmaceutical adjuvant material; preferably, the other therapeutic agents are selected from a chemotherapeutic agent, other antibodies (such as an anti-PD-1 antibody or an anti-PD-L1 antibody) and a cytotoxic agent.

14. A combined product, **characterized in that** the combined product comprises the CLDN18.2 binding molecule of any one of claims 1 to 6 or the immunoconjugate of claim 11, and one or more other therapeutic agents, for example, a chemotherapeutic agent, a cytotoxic agent and other antibodies, such as an anti-PD-1 antibody or an anti-PD-L1 antibody.

15. A method for treating a disease associated with CLDN18.2 in a subject, **characterized in that** the method comprises administering to the subject a therapeutically effective amount of the CLDN18.2 binding molecule of any one of claims 1 to 6, the immunoconjugate of claim 11, the pharmaceutical composition of claim 12 or 13, or the combined product of claim 14, wherein the disease associated with CLDN18.2 is, for example, a cancer that expresses or overexpresses CLDN 18.2, for example, bone cancer, blood cancer, lung cancer, hepatic cancer, pancreatic cancer, esophagus cancer, skin cancer, head and neck cancer, cutaneous or intraocular melanoma, uterine cancer, ovarian cancer, rectal cancer, cancer of the anal region, gastric cancer, colon cancer, breast cancer, prostate cancer, uterine cancer, cancers of sexual organs and reproductive organs, Hodgkin's disease, esophageal cancer, small intestine cancer, cancers of endocrine system, thyroid cancer, parathyroid carcinoma, adrenal cancer, soft tissue sarcomas, bladder cancer, renal cancer, renal cell carcinoma, renal pelvis cancer, central nervous system (CNS) tumour, neuroectodermal cancer, spinal axis tumour, glioma, meningioma, and pituitary adenoma, preferably, the cancer is gastric cancer, pancreatic cancer, esophageal cancer, ovarian cancer or lung cancer.

16. A kit for detecting CLDN18.2 in a sample, **characterized in that** the kit comprises the CLDN18.2 binding molecule of any one of claims 1 to 6, for use in performing the steps of:
(a) contacting the sample with the CLDN18.2 binding molecule of any one of claims 1 to 6; and
(b) detecting the formation of a complex of the CLDN18.2 binding molecule and CLDN18.2; optionally, the CLDN18.2 binding molecule is detectably labelled.
